(19) **Europäisches Patentamt / European Patent Office / Office européen des brevets**

(11) **EP 3 859 329 A1**

(12) # EUROPEAN PATENT APPLICATION
published in accordance with Art. 153(4) EPC

(43) Date of publication:
04.08.2021 Bulletin 2021/31

(21) Application number: 19864717.4

(22) Date of filing: 27.09.2019

(51) Int Cl.:
$G01N\ 33/50^{(2006.01)}$     $G01N\ 27/02^{(2006.01)}$
$G01N\ 33/48^{(2006.01)}$     $G16B\ 40/00^{(2019.01)}$

(86) International application number:
PCT/JP2019/038242

(87) International publication number:
WO 2020/067464 (02.04.2020 Gazette 2020/14)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 28.09.2018 JP 2018183381

(71) Applicant: Tohoku Institute of Technology
Sendai-shi, Miyagi 982-8577 (JP)

(72) Inventors:
• SUZUKI, Ikuro
Sendai-shi, Miyagi 982-8577 (JP)
• ISHIBASHI, Yuto
Sendai-shi, Miyagi 982-8577 (JP)

(74) Representative: Uexküll & Stolberg
Partnerschaft von
Patent- und Rechtsanwälten mbB
Beselerstraße 4
22607 Hamburg (DE)

(54) **METHOD, COMPUTER SYSTEM, AND PROGRAM FOR PREDICTING CHARACTERISTIC OF TARGET**

(57) This method for predicting a characteristic of a target includes: (1) a step of obtaining first data relating to a first object; (2) a step of obtaining second data relating to a second object; (3) a step of identifying a prediction parameter set by performing multivariate analysis of the first data and the second data for all combinations of candidate parameters in a candidate parameter set; and (4) a step of predicting the characteristic on the basis of the prediction parameter set, from target data relating to the target.

FIG.7

EP 3 859 329 A1

**Description**

[Technical Field]

The present invention relates to a method, a computer system, or a program for predicting a property of a target. More specifically, the present invention relates to a method or the like for predicting a property of a target compound.

[Background Art]

An effective method of predicting a property of a target with an unknown property is sought after, regardless of the field.

For example, there is data showing that 34% of agents found to be toxic in clinical trials is toxic to the central nervous system. While researches have been conducted to obtain the activity of a neural network of a human derived neuron or the like with a Micro-Electrode Array (MEA) or the like to study the effect of pharmaceutical products in nonclinical trials (Non Patent Literature 1), an effective evaluation method of toxicity to or efficacy on the central nervous system in nonclinical trials has not been found.

[Citation List]

[Non Patent Literature]

[NPL 1] A. Odawara, H. Katoh, N. Matsuda & I. Suzuki, "Physiological maturation and drug responses of human induced pluripotent stem cell-derived cortical neuronal networks in long-term culture", Scientific Reports volume 6, Article number: 26181 (2016)

[Summary of Invention]

[Technical Problem]

The objective of the present invention is to provide a method for predicting an unknown property of a target such as a target compound.

[Solution to Problem]

The present invention provides, for example, the following items.

(Item 1) A method of predicting a property of a target, comprising the steps of:

(1) obtaining first data for a first subject;
(2) obtaining second data for a second subject;
(3) identifying a prediction parameter set by performing multivariate analysis on the first data and the second data for every combination of each of candidate parameters in a candidate parameter set; and
(4) predicting the property based on the prediction parameter set from target data for the target.

(Item 2) The method of item 1, wherein the step (3) for identifying the prediction parameter set comprises identifying a combination of the candidate parameters that can isolate the first subject from the second subject by the multivariate analysis as the prediction parameter set.
(Item 3) The method of item 1 or 2, wherein the step (4) for predicting comprises comparing the target data with the first data and the second data to identify a property of a subject that yields data that is similar to the target data as a property of the target.
(Item 4) The method of item 3, comprising determining which of the first data and the second data the target data is similar to by multivariate analysis on the target data, the first data, and the second data for the prediction parameter set.
(Item 5) The method of item 4, wherein the similarity is determined by a Euclidian distance, cosine similarity, or a combination thereof.
(Item 6) The method of any one of items 1 to 5, further comprising a step of:
(5) predicting a second property from second target data for the target based on a second prediction parameter set corresponding to the property predicted in the step (4).

(Item 7) The method of any one of items 1 to 6, wherein the multivariate analysis is principle component analysis or cluster analysis.

(Item 8) The method of any one of items 1 to 7, wherein the target is a compound.

(Item 9) The method of item 8, wherein the property comprises one or more of efficacy, toxicity, and mechanism of action of the compound.

(Item 10) The method of item 9, wherein the data is activity data for a neuron.

(Item 11) The method of item 10, wherein the activity data is obtained using one of a micro-electrode array, $Ca^{2+}$ imaging, and membrane potential imaging.

(Item 12) The method of item 10 or 11, wherein the neuron is a neural stem cell.

(Item 13) The method of item 12, wherein the neural stem cell is an iPS cell.

(Item 14) The method of any one of items 10 to 13, wherein the candidate parameter set comprises a basic activity parameter.

(Item 15) The method of item 14, wherein the basic activity parameter comprises TS, NoB, a burst frequency, a burst percentage, and an interquartile range of a burst duration.

(Item 16) The method of any one of items 10 to 15, wherein the candidate parameter set comprises a mean value of burst structure parameters.

(Item 17) The method of any one of items 10 to 15, wherein the candidate parameter set comprises a standard deviation or a median absolute deviation of burst structure parameters.

(Item 18) The method of item 16 or 17, wherein the burst structure parameters comprise a burst duration, a spike count in a burst, IBI, IPI, PS, peak time percentage, mean ISI within a burst, median ISI within a burst, and median/mean ISI within a burst.

(Item 19) The method of any one of items 10 to 18, wherein the candidate parameter set further comprises a parameter related to periodicity.

(Item 20) The method of item 19, wherein the parameter related to periodicity comprises a periodic parameter.

(Item 21) The method of item 19 or 20, wherein the parameter related to periodicity further comprises coefficients of variation of each of a burst duration, a spike count in a burst, IBI, IPI, PS, peak time percentage, mean ISI within a burst, median ISI within a burst, and median/mean ISI within a burst and a mean value of CV ISI within a burst, standard deviation of CV ISI within a burst, median absolute deviation of CV ISI within a burst, and coefficients of variation of CV ISI within a burst.

(Item 22) The method of any one of items 10 to 21, wherein the candidate parameter set comprises a parameter for analyzing a frequency.

(Item 23) The method of item 22, wherein the parameter for analyzing a frequency comprises a parameter for analyzing a frequency of about 250 Hz or less.

(Item 24) The method of any one of items 10 to 23, wherein the candidate parameter set comprises a nonlinear time series analysis parameter.

(Item 25) A method of predicting a property of a target, comprising the steps of:

obtaining data for each of a first subject, a second subject, and the target;

performing multivariate analysis on each data for a prediction parameter set;

determining which of the data for the first subject andc the data for the second subject the data for the target is similar to; and

identifying a property of the first subject as the property of the target if the data for the target is determined to be more similar to the data for the first subject than to the data for the second subject.

(Item 26) The method of item 25, wherein the prediction parameter set comprises one or more of a periodic parameter, a parameter for analyzing a frequency of about 250 Hz or less, and a nonlinear time series analysis parameter.

(Item 27) A computer system for predicting a property of a target, comprising:

means for receiving first data for a first subject and second data for a second subject;

means for performing multivariate analysis on the first data and the second data for every combination of each of candidate parameters in a candidate parameter set;

means for identifying a prediction parameter set based on a result of the multivariate analysis; and

means for predicting the property based on the prediction parameter set from target data for the target.

(Item 28) A program for predicting a property of a target, the program being executed in a computer system comprising a processor, the program causing the processor to execute processing comprising the steps of:

(1) receiving first data for a first subject;

(2) receiving second data for a second subject;

(3) identifying a prediction parameter set by performing multivariate analysis on the first data and the second data for every combination of each of candidate parameters in a candidate parameter set; and

(4) predicting the property based on the prediction parameter set from target data for the target.

(Item 29) A computer system for predicting a property of a target, comprising:

means for receiving data for each of a first subject, a second subject, and the target;

means for performing multivariate analysis on each data for a prediction parameter set;

means for determining which of the data for the first subject and the data for the second subject the data for the target is similar to; and

means for identifying a property of the first subject as the property of the target if the data for the target is determined to be more similar to the data for the first subject than to the data for the second subject.

(Item 30) A program for predicting a property of a target, the program being executed in a computer system comprising a processor, the program causing the processor to execute processing comprising the steps of:

receiving data for each of a first subject, a second subject, and the target;

performing multivariate analysis on each data for a prediction parameter set;

determining which of the data for the first subject and the data for the second subject the data for the target is similar to; and

identifying a property of the first subject as the property of the target if the data for the target is determined to be more similar to the data for the first subject than to the data for the second subject.

[Advantageous Effects of Invention]

The present invention can provide a method or the like of predicting an unknown property (e.g., toxicity to or efficacy on the nervous system) of a target such as a target compound.

[Brief Description of Drawings]

[Figure 1] Figure **1** is a block diagram showing an example of a configuration of a computer system **100** for predicting a property of a target compound of the invention.

[Figure 2] Figure **2** is a block diagram showing an example of a configuration of a processor **120.**

[Figure 3] Figure **3** is a diagram describing examples of candidate parameters contained in a candidate parameter set.

[Figure 4A] Figure **4A** is (a) a diagram combining a raster plot image and a histogram generated from the raster plot image, and (b) a diagram showing a power spectrum deduced by performing discrete Fourier transform (DFT) on the histogram of (a).

[Figure 4B] Figure **4B** is a diagram showing an example of a dominant frequency band.

[Figure 4C] Figure **4C** is a bar graph showing the values of periodicity and NoB when assuming the value prior to administration of a compound as 100%.

[Figure 5A] Figure **5A** is a diagram schematically showing an example of a waveform image decomposed into a plurality of frequency components.

[Figure 5B] Figure **5B** is a graph showing an example of a result of computing the intensity for each frequency component in Figure **5A.**

[Figure 6A] Figure **6A** is an example of principle component plots of a first known compound, second known compound, and target compound.

[Figure 6B] Figure **6B** is a diagram showing an example of a result of performing cluster analysis on first to eleventh known compounds and target compound A and target compound B.

[Figure 7] Figure **7** is a flowchart showing an example of processing in the computer system **100** for predicting a property of a target compound.

[Figure 8] Figure **8** is a flowchart showing an example of processing to predict a property of a target compound by the processor **120** at step **S704.**

[Figure 9] Figure **9** is a flowchart showing an example of processing in the computer system **100** for predicting a property of a target compound.

[Figure 10A] Figure **10A** is a diagram showing a result of predicting a mechanism of action of target compound A in one Example.

[Figure 10B] Figure **10B** is a diagram showing a result of predicting a mechanism of action of target compound A in one Example.

[Description of Embodiments]

The present invention is described hereinafter. The terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

(Definition)

As used herein, "target compound" refers to a compound targeted for prediction of a property. A target compound can be an unknown compound or a known compound. Examples of properties of a target compound include, but are not limited to, efficacy, toxicity, and mechanism of action.

As used herein, "efficacy" refers to an effect induced as a result of applying an agent to a subject. If an agent is for example an anti-cancer agent, the efficacy can be a direct effect on a subject such as contraction of a cancerous area under X-ray observation, delay in advancement of cancer, and extended survival of a cancer patient, or an indirect effect such as a reduction in a biomarker correlated with advancement of cancer. As used herein, "efficacy" is intended as an effect under any application condition. If an agent is for example an anti-cancer agent, the efficacy can be an effect in a specific subject (e.g., 80-year-old or older male) or an effect under a specific application condition (e.g., under concomitant use with another anticancer therapy). In one embodiment, an agent can have a single efficacy or a plurality of efficacies. In one embodiment, an agent can have efficacy that varies under different application conditions. In general, efficacy refers to an effect intended to be achieved.

As used herein, "toxicity" refers to an unfavorable effect that is manifested from application of an agent to a subject. In general, toxicity is an effect that is different from the intended effect of an agent. Toxicity can manifest through the same or different mechanism of action from efficacy. If an agent is for example an anti-cancer agent, hepatotoxicity due to killing of normal hepatocytes may manifest concurrently with the efficacy for killing cancer cells via the mechanism of action of suppression of cell proliferation, or toxicity for a neurological dysfunction may manifest via the mechanism of action of membrane stabilization concurrently with efficacy for killing cancer cells via the mechanism of action of suppression of cell proliferation.

As used herein, "mechanism of action" refers to the manner of interaction of an agent with a biological mechanism. If an agent is for example an anti-cancer agent, the mechanism of action can be various levels of events, such as activation of the immune system, killing of cells with a rapid growth rate, blockage of proliferative signaling, blockage of a specific receptor, and inhibition of transcription of a specific gene. If the mechanism of action is identified, efficacy, toxicity, and/or suitable usage form can be predicted based on accumulated information.

As used herein, "about" refers to a range of ± 10% from the numerical value that is described subsequent to "about".

1. Prediction of property of target compound

The inventors of the present invention developed an approach of predicting a property of a target compound using multivariate analysis in order to evaluate a property of a target compound with respect to the nervous system in non-clinical trials. The method of the invention can deduce a prediction parameter set that is suitable for isolating a response of a known compound by using multivariate analysis on multiple (preferably all) combinations in a parameter set comprising a plurality of candidate parameters. Activity data obtained when a target compound is administered to a neuron is analyzed using the prediction parameter set to predict the known compound whose property is similar to a property of the target compound.

For example, activity data obtained when each of a plurality of known compounds having each of the properties to be isolated (e.g., when isolating the property of "presence/absence of toxicity", known compound A with a property of "with toxicity", and known compound B with a property of "without toxicity") is administered to a neuron is subjected to multivariate analysis to deduce a prediction parameter set that can isolate such properties. It is possible to predict whether a property of a target compound is "with toxicity" or "without toxicity" by analyzing activity data obtained when the target compound is administered to a neuron by using such a prediction parameter set. Deduction of a prediction parameter set in accordance with a property to be isolated enables the prediction of what efficacy the target compound has, what toxicity the target compound has, or what mechanism of action the target compound has. This enables prediction of efficacy, toxicity, or mechanism of action of a target compound in nonclinical trials. For example, analysis using human iPS cells enables prediction of the action or toxicity of a compound on the human nervous system, leading to materialization of rapid and cost effective development of a pharmaceutical product or development of compounds in general.

Furthermore, this methodology can also predict and output, for example, what mechanism of action a target compound has for the predicted efficacy or what mechanism of action a target compound has for the predicted toxicity. In this manner, not only abstract properties, but also specific properties of a target compound can be predicted.

Furthermore, if a known compound is used as a target compound, drug repositioning of the known compound can be performed. Specifically, a new efficacy of the known compound can be found based on the efficacy, toxicity, or mechanism of action predicted by analyzing activity data obtained when the known compound is administered to a neuron. This enables discovery of new efficacy of the known compound and drastic reduction in development cost of a therapeutic drug in nonclinical trials.

Further, the deduced prediction parameter set would not be dependent on a difference between cell species, difference between samples, or difference between facilities by using data with a difference between cell species, difference between samples, or difference between facilities when deducing a prediction parameter set. This allows construction of a consistent agent evaluation system using neural activity data.

Such a prediction of a property of a target compound using multivariate analysis can be materialized, for example, with a computer system for predicting a property of a target compound described below.

The embodiments of the invention are described hereinafter with reference to the drawings.

2. Configuration of a computer system for predicting a property of a target compound

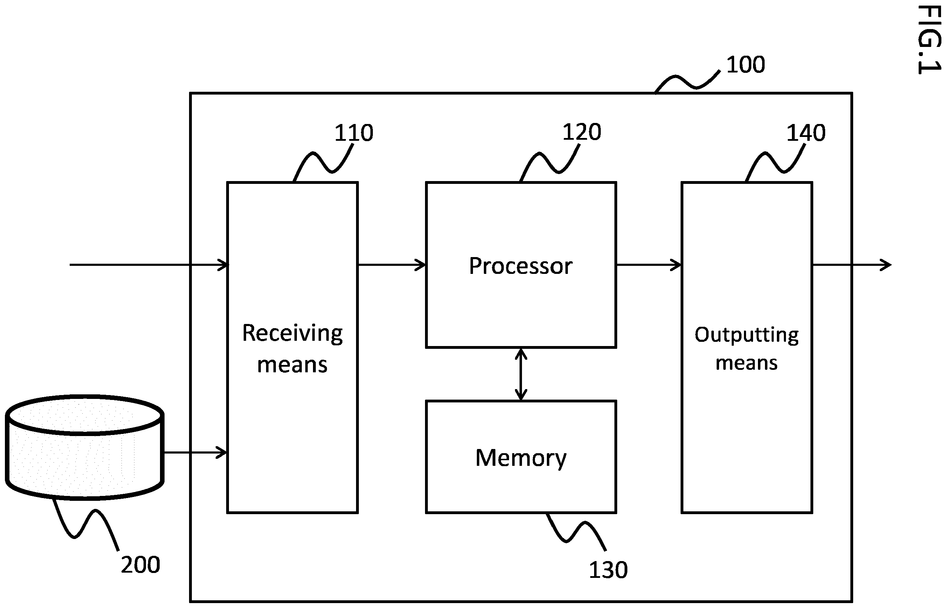

Figure **1** shows an example of a configuration of the computer system **100** for predicting a property of a target compound of the invention.

The computer system **100** comprises receiving means **110,** a processor **120,** a memory **130,** and outputting means **140.** The computer system **100** can be connected to a database unit **200.**

The receiving means **110** is configured to be able to receive data from the outside of the computer system **100.** The receiving means **110** can, for example, receive data via a network from the outside of the computer system **100,** or receive data from a storage medium (e.g., USB memory, optical disk, or the like) or the database unit **200** connected to the computer system **100.** When receiving data via a network, the type of network is not limited. The receiving means **110** can, for example, receive data by utilizing a wireless LAN such as Wi-Fi or receive data via the Internet.

For example, the receiving means **110** receives activity data for a neuron in response to a known compound or target compound. Activity data for a neuron is data that can be obtained by any known methodology.

Activity data for a neuron is, for example, potential data measured using a micro-electrode array (MEA). Such potential data is obtained, for example, by culturing a neuron on an MEA and measuring the activity potential and synaptic current component of the neuron upon administration of a known compound or target compound to the cultured neuron. For example, a CMOS-MEA utilizing CMOS can be used as the MEA. When CMOS-MEA is used, relatively high resolution data can be obtained.

Activity data for a neuron is, for example, potential data measured using a multipoint electrode. Such potential data is obtained, for example, by applying a multipoint electrode on the brain of an animal and measuring the activity potential of a neuron when a known compound or target compound is administered in an animal experiment.

Activity data for a neuron can also be, for example, image data obtained by an optical measurement method. An optical measurement method is, for example, $Ca^{2+}$ imaging. For example, image data is obtained by imaging the activity of a neuron when a known compound or target compound is administered to the neuron through $Ca^{2+}$ imaging. An optical measurement method is, for example, membrane potential imaging using a membrane potential sensitive dye. For example, image data is obtained by imaging the activity of a neuron when a known compound or target compound is administered to the neuron through membrane potential imaging.

In one embodiment, use of MEA can be preferable from the viewpoint of temporal resolution. This is because MEA has higher temporal resolution and is capable of more accurately detecting each spike, so that the amount of information obtained on activity data is greater, compared to $Ca^{2+}$ imaging and membrane potential imaging. Membrane potential imaging has higher temporal resolution than $Ca^{2+}$ imaging.

In another embodiment, use of MEA can be preferable from the viewpoint of long-term measurement. This is because MEA is less invasive compared to $Ca^{2+}$ imaging and membrane potential imaging.

In still another embodiment, use of $Ca^{2+}$ imaging or membrane potential imaging can be preferable from the viewpoint of cell resolution. This is because $Ca^{2+}$ imaging or membrane potential imaging can mark all cells through microscope observation, so that spatial resolution is higher compared to MEA and therefore raster plot images can be readily created in subsequent processing.

The receiving means **110** can be configured, for example, to receive an image from converting activity data for a neuron in response to a known compound or a target compound. A converted image can be, for example, a raster plot image from converting activity data for a neuron obtained as potential data or activity data for a neuron obtained as an image data, a histogram from converting activity data for a neuron obtained as potential data or activity data for a neuron obtained as an image data, a waveform image converted from activity data for a neuron obtained as potential data, or a frequency intensity map or spectrum intensity map from converting activity data for a neuron obtained as potential data by frequency component analysis (e.g., wavelet transform or Fourier transform (e.g., fast Fourier transform (FFT))). A spectrum intensity map can be obtained, for example, by applying FFT on a waveform obtained by converting activity data for a neuron obtained as potential data into a waveform (or frequency division waveform) and dividing the waveform by a predetermined time interval (e.g., one second interval). In this regard, a frequency division waveform refers to a waveform from extracting a specific frequency band component. A specific frequency band component can be, for example, a $\theta$ wave frequency component (about 4 to about 8 Hz), $\alpha$ wave frequency component (about 8 to about 13 Hz), $\beta$ wave frequency component (about 13 to about 30 Hz), $\gamma$ wave frequency component (about 30 to about 100 Hz), or the like. In one embodiment, a frequency band component can be a $\theta$ wave frequency component. In another embodiment, a frequency band component can be an $\alpha$ wave frequency component. In still another embodiment, a frequency band component can be a $\beta$ wave frequency component. In still another embodiment, a frequency band component can be a $\gamma$ wave frequency component. Use of a frequency division waveform enables analysis focusing on a phenomenon observed in vivo. For example, a $\gamma$ wave frequency component of brain waves is enhanced upon an epileptic seizure, so that a property of a target compound associated with epileptic seizure can be analyzed by using a frequency division waveform of a $\gamma$ wave frequency component.

In this regard, a neuron is, for example, a neural stem cell. A neural stem cell can be, for example, an animal neural stem cell or a human neural stem cell. A neural stem cell is, for example, an iPS cell. A neuron can be, for example, a primary neuron harvested from an animal.

Data received by the receiving means **110** is passed along to the processor **120** for subsequent processing.

The processor **120** controls the entire operation of the computer system **100**. The processor **120** reads out a program stored in the memory **130,** and executes the program. This allows the computer system **100** to function as an apparatus for executing a desired step. The processor **120** can be implemented by a single processor or a plurality of processors. Data processed by the processor **120** is passed along to the outputting means **140** for output.

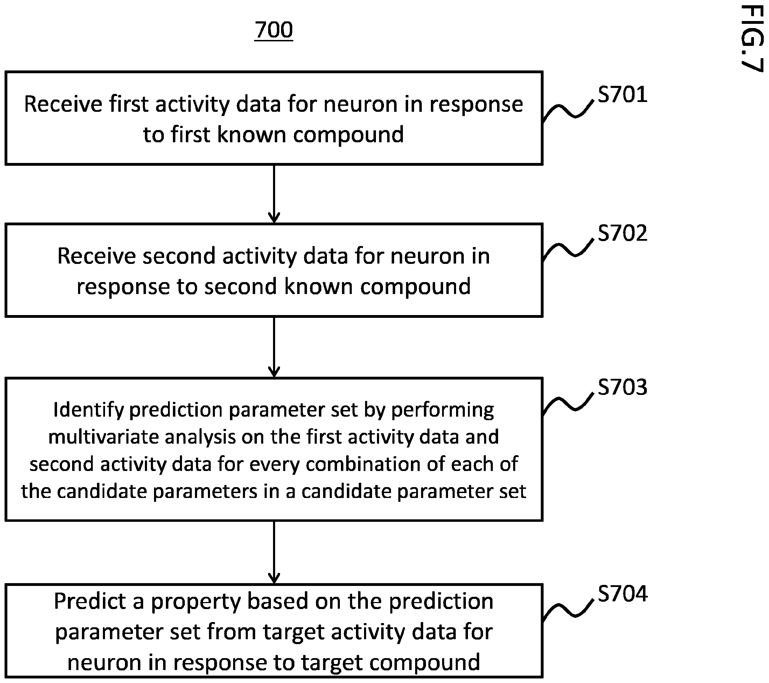

In the memory **130,** a program for executing processing in the computer system **100,** data required for executing the program, and the like are stored. For example, a program for predicting a property of a target compound (e.g., program for materializing the processing shown in Figure **7, 8,** or **9** described below) is stored in the memory **130.** An application implementing any function can be stored in the memory **130.** For example, a program for converting received potential data or image data into a raster plot image, histogram, waveform image, or the like can be stored. In this regard, how a program is stored in the memory **130** is not limited. For example, a program can be preinstalled in the memory **130.** Alternatively, a program can be installed in the memory **130** by being downloaded via a network. The memory **130** can be implemented with any storage means.

The outputting means **140** is configured to be able to output data to the outside of the computer system **100.** The mode of how the outputting means **140** is enabled to output information from the computer system **100** is not limited. If the outputting means **140** is, for example, a display screen, information can be outputted on the display screen. Alternatively, if the outputting means **140** is a speaker, information can be outputted via audio from the speaker. Alternatively, if the outputting means **140** is a data writing apparatus, information can be outputted by writing information into a storage medium or the database unit **200** connected to the computer system **100.** Alternatively, if the outputting means **140** is a transmitter, data can be outputted by the transmitter sending information out of the computer system **100** via a network. In such a case, the type of network is not limited. For example, a transmitter can transmit information via the Internet or via LAN. For example, the outputting means **140** can output data after converting the data into a format that is compatible with the hardware or software receiving the output, or after adjusting the data to a response rate that is compatible with the hardware or software receiving the output.

For example, activity data for a neuron in response to a known compound can be stored in the database unit **200** connected to the computer system **100.** Activity data for a neuron in response to a known compound can be stored while being associated with a property of the known compound. For example, activity data for a neuron in response to a target compound can be stored in the database unit **200.** For example, data outputted by the computer system **100** (e.g., predicted property of a target compound) can be stored in the database unit **200.** An identified prediction parameter can be stored in the database unit **200.** Data resulting from multivariate analysis on activity data for a neuron in response to a known compound and/or activity data for a neuron in response to a target compound can be stored in the database unit **200.** Activity data for a neuron in response to a known compound or target compound stored in the database unit **200** can be stored, for example, after removing data representing an outlier value. This can be accomplished, for example, by utilizing a property of a compound that can be identified by the multivariate analysis described below to exclude activity data that does not have the same property as such a property as an outlier value. This can refine data that can be stored in the database unit **200.**

In the example illustrated in Figure **1,** the database unit **200** is provided external to the computer system **100,** but the present invention is not limited thereto. The database unit **200** can also be provided inside the computer system **100.** In such a configuration, the database unit **200** can be implemented by the same or different storage means as the storage means implementing the memory **130.** In either configuration, the database unit **200** is configured as a storage unit for the computer system **100.** The configuration of the database unit **200** is not limited to a specific hardware configuration. For example, the database unit **200** can be comprised of a single hardware part or a plurality of hardware parts. For example, the database unit **200** can be configured as an external hard disk drive of the computer system **100,** or as a storage on the cloud connected via a network.

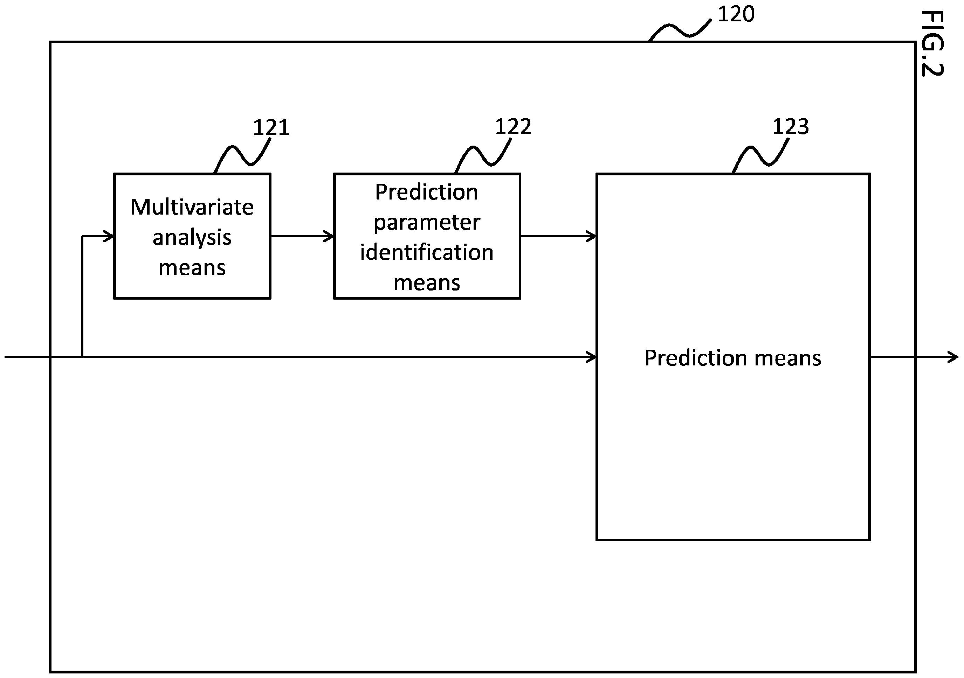

Figure **2** shows an example of the configuration of the processor **120.**

The processor **120** comprises at least multivariate analysis means **121,** prediction parameter identification means **122,** and prediction means **123.**

The multivariate analysis means **121** is configured to perform multivariate analysis on inputted data. For example, the multivariate analysis means **121** performs multivariate analysis on received activity data for a neuron in response to a first known compound and activity data for a neuron in response to a second known compound. Multivariate analysis is, for example, principle component analysis. At least the first principle component and second principle component of data can be computed by principle component analysis. For example, principle component analysis can be configured to compute the third principle component, fourth principle component ... n-1st principle component (wherein n is the number of dimensions of inputted data). Multivariate analysis is, for example, cluster analysis. Data can be classified into a plurality of clusters by cluster analysis. For example, Ward's method (internal square distance) can be used as the algorithm of cluster analysis. The mean distance, distance between centroids, maximum distance, or minimum distance can also be used. For example, the Euclidean distance can be used as an indicator for determining similarity. The Mahalanobis distance or Spearman's rank correlation can also be used. For example, cosine similarity can also be used as an indicator for determining similarity. For example, a combination of Euclidean distance and cosine similarity can also be used as an indicator for determining similarity. A threshold value of an indicator for determining similarity can be set to any value in accordance with the data.

Data inputted into the multivariate analysis means **121** can comprise, for example, a plurality of pieces of data from administering the same compound to different cell species, a plurality of pieces of data from administering the same compound to the same cell species supplied by different vendors, a plurality of pieces of data from administering the same compound to samples of different lots supplied by the same vendor, a plurality of pieces of data from administering the same compound to samples supplied on different dates by the same vendor, or a plurality of pieces of data from testing the same compound at different facilities. In this manner, a prediction parameter set that is not dependent on the difference between samples, difference between cell species, or difference between facilities can be identified with the prediction parameter identification means **122** described below by processing a plurality of pieces of data with the multivariate analysis means **121.**

Data inputted into the multivariate analysis means **121** can be, for example, activity data for a neuron in response to a known compound or a target compound or an image converted therefrom. A converted image can be, for example, a raster plot image from converting activity data for a neuron obtained as potential data or activity data for a neuron obtained as an image data, a histogram from converting activity data for a neuron obtained as potential data or activity data for a neuron obtained as an image data, a waveform image converted from activity data for a neuron obtained as potential data, or a frequency intensity map from converting activity data for a neuron obtained as potential data by frequency component analysis (e.g., wavelet transform). A converted image can be, for example, an image received by the receiving means **110** or an image converted by the processor **120** in a stage prior to the multivariate analysis means **121.**

For example, the multivariate analysis means **121** performs multivariate analysis on activity data for a neuron in response to a first known compound and activity data for a neuron in response to a second known compound for every combination of each of the candidate parameters in a candidate parameter set. The multivariate analysis means **121** can perform multivariate analysis on activity data for a neuron in response to three or more compounds for every combination of each of the candidate parameters in a candidate parameter set.

A parameter set for multivariate analysis needs to comprise three or more candidate parameters because multivariate analysis cannot be performed with two or fewer candidate parameters. If, for example, a candidate parameter set consists of 10 candidate parameters, every combination of each of the candidate parameters would be 968 parameter sets (each parameter set comprising 3 to 10 candidate parameters). In this regard, the multivariate analysis means **121** performs multivariate analysis on activity data for a neuron in response to a first known compound and activity data for a neuron in response to a second known compound for each of the 968 parameter sets.

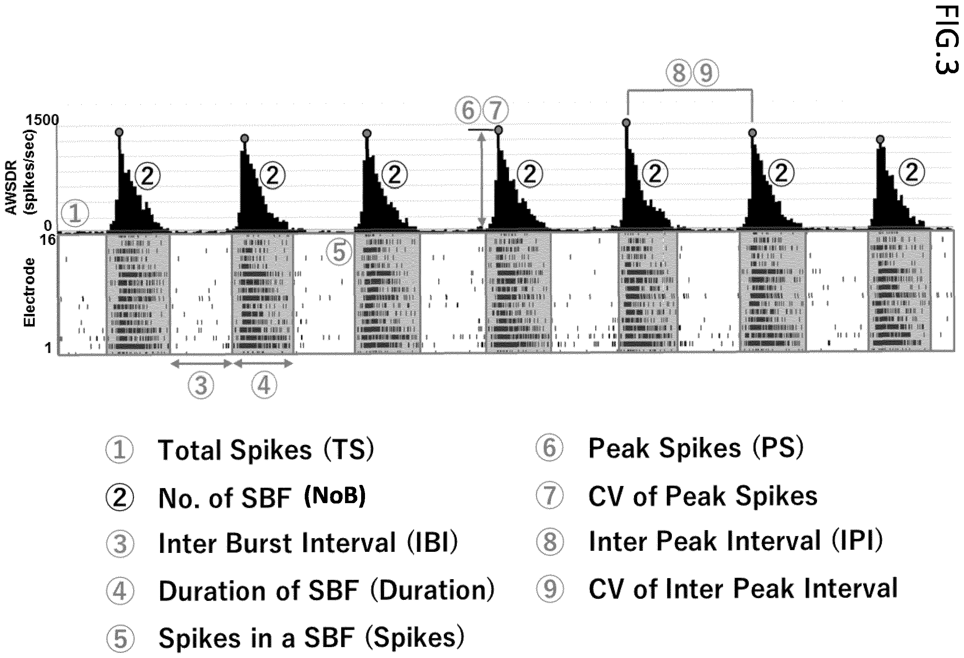

Figure **3** is a diagram describing an example of candidate parameters contained in a candidate parameter set.

The graph in Figure **3** is a diagram combining a raster plot image and a histogram generated from the raster plot image. The diagram shows a raster plot image on the bottom side of the graph, and a histogram on the top side of the graph. In the raster plot image, the vertical axis indicates an electrode, the horizontal axis indicates time, and black plots represent spikes. In the histogram, the vertical axis indicates the spike count per second, and the horizontal axis indicates time.

Examples of candidate parameters include:

(1) Total Spikes (TS: total number of spikes detected during measurement);
(2) Number of SBF (NoB: total number of synchronized burst firing detected during measurement);
(3) Inter Burst Interval (IBI: time from the end of synchronized burst firing to the start of the next synchronized burst firing);
(4) Duration of SBF (burst duration: time from start to end of synchronized burst firing);
(5) Spikes in a SBF (spike count in burst: spike count detected in synchronized burst firing);
(6) Peak Spikes (PS: peak value of spike count in a histogram);
(7) CV of Peak Spikes (coefficient of variation of PS; coefficient of variation (CV) = standard deviation/mean value);
(8) Inter Peak Interval (IPI: time from peak value to next peak value in a histogram); and
(9) CV of Inter Peak Interval (coefficient of variation of IPI) .

(Basic activity parameter)

A candidate parameter comprises, for example, a basic activity parameter. A basic activity parameter comprises:

*TS;
*NoB;
*Burst Frequency (NoB/measurement time (s));
*Burst percentage (spike count during all synchronized burst firing/TS); and
*Burst Duration Interquartile Range (interquartile range of burst duration).

By including a basic activity parameter, especially a parameter such as TS or NoB in a candidate parameter set, multivariate analysis is performed while including a concept of increase/decrease in neural network activity. Thus, an effect of being able to isolate a response to a compound using a basic change and directionality in neural network activity due to administration of the compound is attained. A basic activity parameter can be effective, and also essential, for isolating a compound found to have a significant compound response in the increase/decrease in the number of firing, number of burst firing, or the like. Including a basic activity parameter in a candidate parameter set also has an advantage of being able to improve the accuracy of isolating a known compound and the predicting a target compound and prevent an incorrect prediction.

(Burst structure parameter)

A candidate parameter comprises, for example, a mean value of burst structure parameters for all bursts. A burst structure parameter is a parameter related to synchronized burst firing, and includes for example,

*burst duration,
*spike count in a burst,
*IBI,
*IPI,
*PS,
*Peak time percentage (value representing the time at which a peak spike within a burst appears by % when assuming burst duration as 100%; the value is 0% if a peak spike appears at the start time of a burst, and 100% if a peak spike appears at the end time of a burst),
*mean ISI within Burst (mean value of ISI (Inter spike interval: spike interval) within a burst),
*median ISI within Burst (median value of ISI within a burst), and
*median/mean ISI within Burst (median value/mean value of ISI within a burst).

By including a burst structure parameter in a candidate parameter set, multivariate analysis is performed to isolate a response to compound found to have a change in a burst structure parameter. Thus, an effect of detecting a dose dependent change of a compound, isolating a response to a compound, and improving the accuracy of prediction of a mechanism of action is attained.

A candidate parameter can comprise a standard deviation or median absolute deviation of burst structure parameters (e.g., at least one of burst duration, spike count in a burst, IBI, IPI, PS, peak time percentage, mean ISI within burst, median ISI within burst, and median/mean ISI within burst) for all bursts. A candidate parameter can comprise, for example, a median value of burst structure parameters (e.g., at least one of burst duration, spike count in a burst, IBI, IPI, PS, peak time percentage, mean ISI within burst, median ISI within burst, and median/mean ISI within burst) for all bursts. A candidate parameter can comprise, for example, a standard error of burst structure parameters (e.g., at least one of burst duration, spike count in a burst, IBI, IPI, PS, peak time percentage, mean ISI within burst, median ISI within burst, and median/mean ISI within burst) for all bursts.

(Parameter related to periodicity)

A candidate parameter comprises a parameter related to periodicity. A parameter related to periodicity comprises, for example, periodicity (periodicity parameter). Periodicity (periodicity parameter) is represented by the following equation.

[Numeral 1]

$$Periodicity = \frac{All\ frequency\ band}{Dominant\ frequency\ band}$$

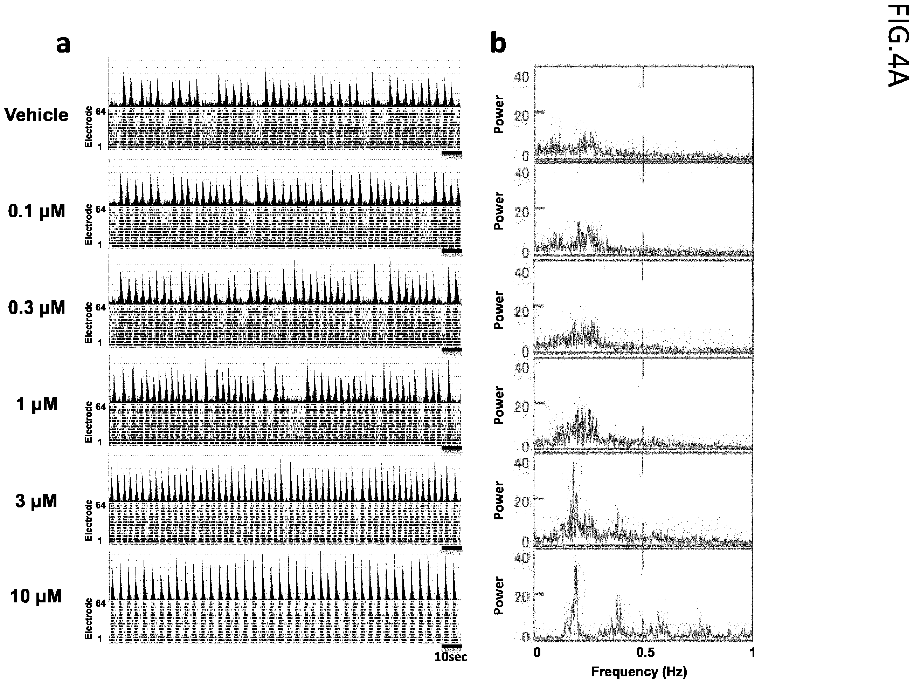

wherein All frequency band is the entire bandwidth of frequencies contained when frequency analysis is performed on a histogram of spike count, such as about 5 Hz. Dominant frequency band is the bandwidth of frequencies at which power exceeds a threshold value when frequency analysis is performed on a histogram of spike count. Periodicity (periodicity parameter) is deduced, for example, as shown in Figures **4A** to **4C.**

Figure **4A-a** is a diagram combining a raster plot image and a histogram generated from the raster plot image. The diagram shows a raster plot image on the bottom side of the graph, and a histogram on the top side of the graph. In the raster plot image, the vertical axis indicates an electrode, the horizontal axis indicates time, and black plots represent spikes. In the histogram, the vertical axis indicates the spike count per second, and the horizontal axis indicates time. The graphs are, from the top, a raster plot image and histogram when administering a vehicle, a raster plot image and histogram when administering a compound at about 0.1 $\mu$M, a raster plot image and histogram when administering a compound at about 0.3 $\mu$M, a raster plot image and histogram when administering a compound at about 1 $\mu$M, a raster plot image and histogram when administering a compound at about 3 $\mu$M, and a raster plot image and histogram when administering a compound at about 10 $\mu$M.

Figure **4A-b** shows a power spectrum deduced by performing discrete Fourier transform (DFT) on the histogram of Figure **4A-a.** The vertical axis indicates power, and the horizontal axis indicates frequency. The graphs are, from the top, a power spectrum when administering a vehicle, a power spectrum when administering a compound at about 0.1 $\mu$M, a power spectrum when administering a compound at about 0.3 $\mu$M, a power spectrum when administering a compound at about 1 $\mu$M, a power spectrum when administering a compound at about 3 $\mu$M, and a power spectrum when administering a compound at about 10 $\mu$M.

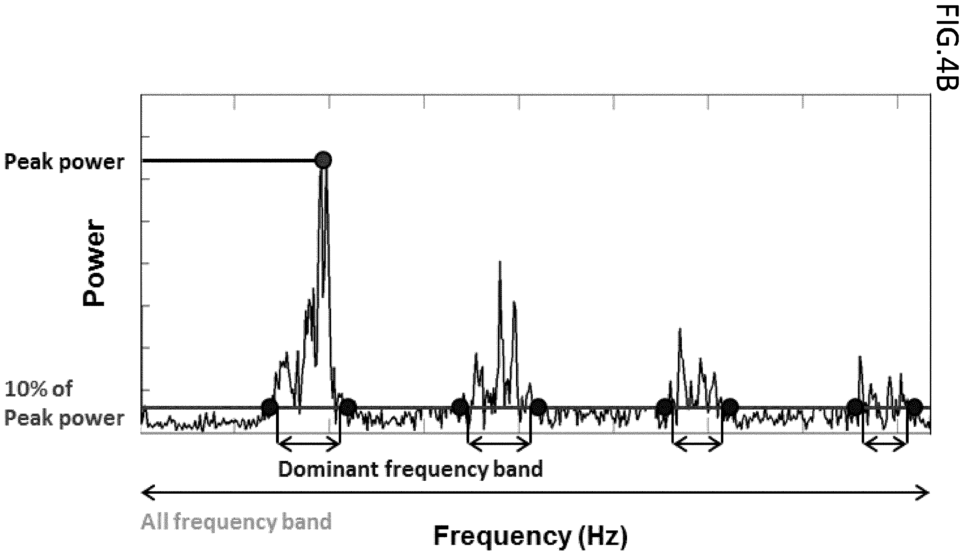

In each power spectrum of Figure **4A-b,** the bandwidth of a frequency band exceeding a threshold value, which is about 10% of a peak value of a basic wave, is defined as the dominant frequency band, as shown in Figure **4B,** and the entire bandwidth of frequencies contained in a power spectrum is defined as the all frequency band. Periodicity can be computer thereby in each power spectrum in Figure **4A-b.** If, for example, a plurality of bandwidths exceed the threshold value as shown in Figure **4B,** the value of the dominant frequency band is the total value of the plurality of bandwidths.

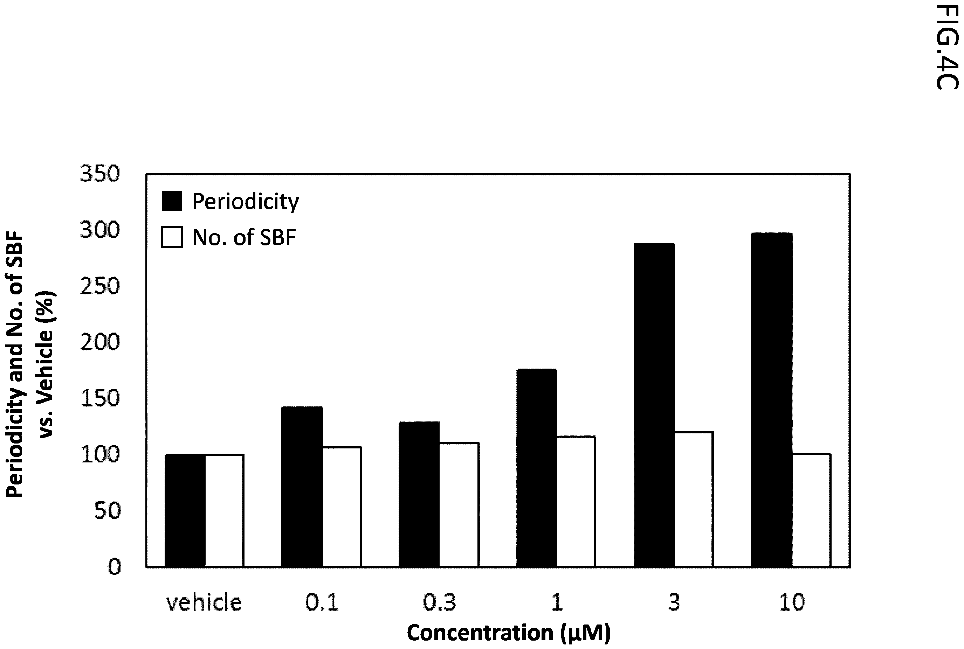

Figure **4C** is a bar graph showing the values of periodicity and NoB when assuming the value from administering a vehicle as 100%. A black bar graph indicates the value of periodicity, and a white bar graph indicates the value of NoB. As can be seen from the graph of Figure **4C,** the value of NoB hardly changes in accordance with the compound concentration, whereas the value of periodicity changes significantly in accordance with the compound concentration. In other words, the value of periodicity can be considered as an indicator reflecting a change in concentration.

A parameter related to periodicity can comprise, for example, a coefficient of variation (CV) of burst structure parameters (e.g., at least one of burst duration, spike count in a burst, IBI, IPI, PS, peak time percentage, mean ISI within burst, median ISI within burst, and median/mean ISI within burst). By including CV of a burst structure parameter in a candidate parameter set, multivariate analysis is performed while including a concept of regularity of the general shape of a burst. Thus, an effect of isolating a known compound with varying regularity in the general shape of a burst and improving the accuracy of prediction of a target compound is attained.

A parameter related to periodicity can comprise, for example, a mean value, standard deviation, standard error, median value, median absolute deviation, or coefficient of variation for all bursts of CV ISI within Burst (coefficient of variation of ISI within burst). Since the coefficient of variation of ISI is computed for each burst for each of these parameters, the mean value, standard deviation, median absolute value, or coefficient of variation for all bursts is computed using a coefficient of variation of ISI for each burst. By including a coefficient of variation of ISI within burst in a candidate parameter set, multivariate analysis is performed while including a concept of regularity of firing within a burst. Thus, an effect of isolating a known compound with varying regularity in firing within a burst and improving the accuracy of prediction of a target compound is attained.

By including the parameter related to periodicity described above in a candidate parameter, multivariate analysis is performed to isolate an agent response found to have periodicity in the occurrence of burst firing. Thus, an effect of detecting a dose dependent change for a compound, isolating a response to a compound, and improving the accuracy of prediction of the mechanism of action is attained. For example, a parameter related to periodicity is effective in isolating the type of compound that excites the neural network activity (seizure positive compound). Inclusion of a parameter related to periodicity in a candidate parameter set also has the advantage of being able to increase types of compounds that can be isolated.

(Parameter for analyzing a frequency)

A candidate parameter comprises, for example, a parameter for analyzing a frequency. A parameter for analyzing a frequency is, for example, a parameter that can analyze a frequency component of about 250 Hz or less. A frequency component of about 250 Hz or less is less than an action potential component (about 1 kHz or greater) and is reflective of a post synaptic current component that is important for a neurological function. Thus, a property of a compound with respect to a neuron can be detected with a parameter for analyzing a frequency component of about 250 Hz or less. Furthermore, a parameter for analyzing a frequency component of about 250 Hz or less is an indicator that can be compared with in vivo brain waves and allows correlation between in vivo and in vitro frequencies to be measured.

A parameter for analyzing a frequency comprises, for example, intensity computed from potential data or waveform image. Such an intensity decomposes potential data or waveform image into a plurality of frequency components, and is represented by the following equation in terms of each of the decomposed frequency component.

[Numeral 2]

$$\text{Intensity} = \frac{\sum(|\text{action potential}| \times \text{sampling period})}{\text{synchronized burst firing period}}$$

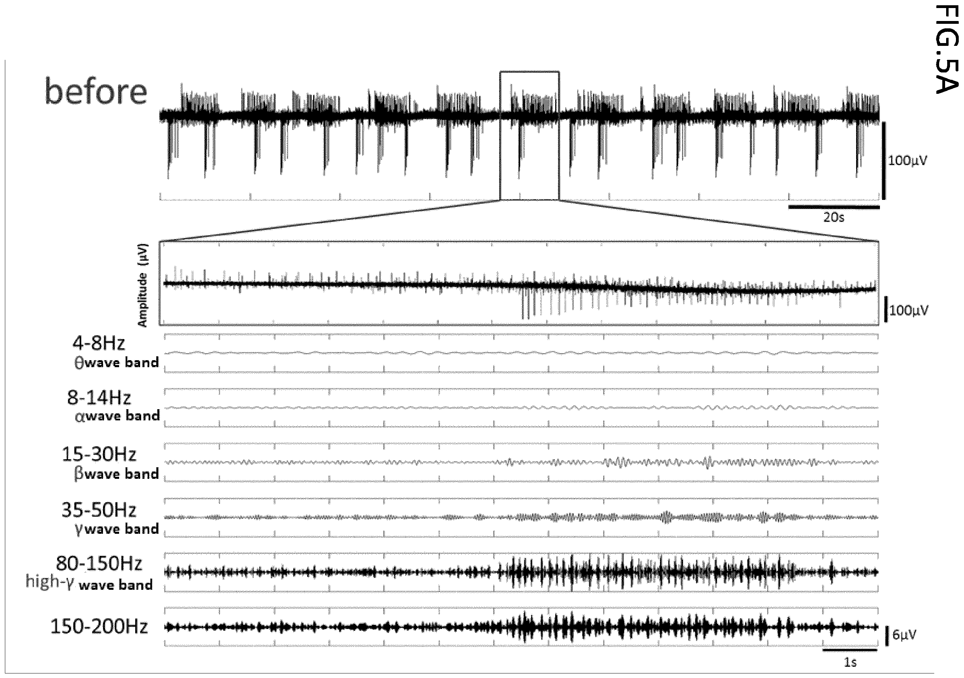

A plurality of frequency components include, for example, a $\theta$ wave band of about 4 to about 8 Hz, $\alpha$ wave band of about 8 to about 14 Hz, $\beta$ wave band of about 15 to about 30 Hz, $\gamma$ wave band of about 35 to about 50 Hz, high-$\gamma$ wave band of about 80 to about 150 Hz, and about 150 to about 200 Hz. A waveform image is decomposed into a plurality of frequency components as shown in Figure **5A.** Decomposition is performed, for example, by using a band pass filter corresponding to each frequency component.

Figure **5A** schematically shows an example of a waveform image decomposed into a plurality of frequency components. The graph shown in Figure **5A** is a potential waveform image. The vertical axis indicates potential, and the horizontal axis indicates time.

In the example shown in Figure **5A,** a periodic waveform comprising a single burst is extracted and decomposed into frequency component of a $\theta$ wave band of about 4 to about 8 Hz, $\alpha$ wave band of about 8 to about 14 Hz, $\beta$ wave band of about 15 to about 30 Hz, $\gamma$ wave band of about 35 to about 50 Hz, high-$\gamma$ wave band of about 80 to about 150 Hz, and about 150 to about 200 Hz.

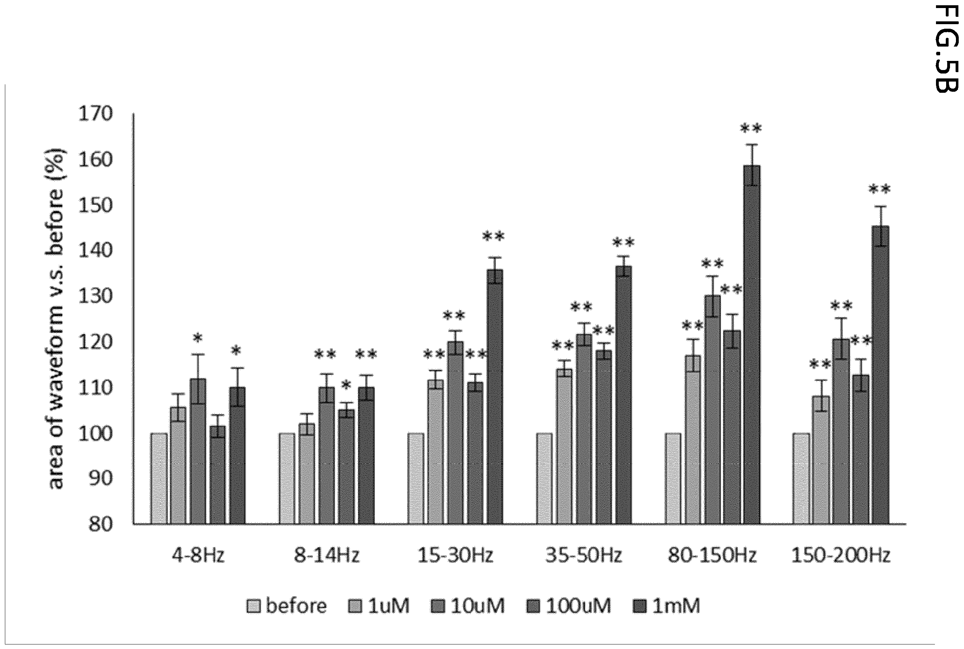

Figure **5B** is a graph showing an example of a result of computing the intensity for each frequency component in Figure **5A.** The vertical axis indicates the value of intensity when assuming the value prior to administration of the compound as 100%. Figure **5B** shows results at a plurality of concentrations (about 1 $\mu$M, about 10 $\mu$M, about 100 $\mu$M, and about 1 mM). As can be seen from the graph of Figure **5B,** the value of intensity changes in accordance with the compound concentration for each frequency component. Specifically, the value of intensity can be considered as an indicator reflecting the change in concentration.

A parameter for analyzing a frequency comprises, for example, intensity computed from wavelet transform. Such intensity is computed from a scalogram generated by waveform transform of potential data or waveform image. For example, intensity can be computed by finding the difference between a scalogram generated by wavelet transform on data prior to administration of a compound and a scalogram generated by wavelet transform on data after administration of the compound.

In a scalogram generated by wavelet transform, the pixel count of the X axis varies by the burst duration, and the pixel count of the Y axis is for example 170 pixels. The pixel count of the Y axis occupying each frequency band varies by each frequency band. For example, the pixel count of the Y axis of a frequency band of about 5 to about 8 Hz is 21 pixels, the pixel count of the Y axis of a frequency band of about 8 to about 14 Hz is 15 pixels, the pixel count of the Y axis of a frequency band of about 15 to about 25 Hz is 24 pixels, the pixel count of the Y axis of the frequency band of about 30 to about 50 Hz is 31 pixels, the pixel count of the Y axis of the frequency band of about 70 to about 150 Hz is 34 pixels, and the pixel count of the Y axis of the frequency band of about 150 to about 200 Hz is 13 pixels. Thus, the intensity of each frequency band cannot be simply compared. However, the intensity of each frequency can be compared after standardization using the following equation.

[Numeral 3]

$$WT_A = \frac{WT_S}{(N_X \times N_Y(f))}$$

wherein $WT_A$ is a wavelet transform coefficient per pixel of each frequency band, $WT_S$ is the total value of wavelet transform coefficients of each frequency band, $N_X$ is the pixel count of the X axis, and $N_Y(f)$ is the pixel count of the Y axis of each frequency band. Intensity can be computed by deriving $WT_A$ from a scalogram generated by wavelet transform on data prior to administration of the compound, deriving $WT_A$ from a scalogram generated by wavelet transform on data after administration of the compound, and finding the difference between the $WT_A$ prior to administration of the compound and the $WT_A$ after administration of the compound.

A parameter for analyzing a frequency comprises, for example, spectrum intensity computed by FFT. Such spectrum intensity is computed from a spectrum intensity map generated by fast Fourier transform (FFT) on a waveform obtained by converting potential data into a waveform and dividing the waveform by a predetermined time interval (e.g., one second interval). For example, the spectrum intensity of each frequency for each 1 Hz from 1 to 250 Hz can be computed from a spectrum intensity map generated by FFT on a waveform. Such spectrum intensities can be utilized as 250 parameters. Furthermore, a chronological change in multivariate analysis results can be analyzed by utilizing spectrum intensity in a time series using waveforms within a plurality of time intervals. This enables isolation of a known compound and prediction of a target compound based on a chronological change.

(Nonlinear time series analysis parameter)

A candidate parameter comprise, for example, a nonlinear time series analysis parameter. A nonlinear time series analysis parameter is, for example, a scaling index $\alpha$ computed by Detrended fluctuation analysis (DFA). DFA is a methodology for eliminating non-stationary variable components (trend) and characterizing long-range correlation in fluctuations with a scaling index $\alpha$. Variation $F(n)$ computed by DFA has the relationship of

[Numeral 4]

$$F(n) \sim n^\alpha$$

wherein scaling index $\alpha$ of 0.5 indicates no long-range correlation, $\alpha > 0.5$ indicates a positive correlation, $\alpha < 0.5$ indicates a negative correlation, and $\alpha = 1$ indicates 1/f noise.

For example, time series ISI can be quantified with scaling index $\alpha$ by performing DFA on the time series ISI while focusing on the regularity of the time of firing.

A candidate parameter can comprise, for example, a Z score of the firing interval between electrodes. Z score is an indicator of synaptic connection strength of cells. Z score is computed, for example, by the following equation.

[Numeral 4-1]

$$Z\ score = \frac{N_{real} - Ave_{surrogate}}{SD_{surrogate}}$$

wherein $N_{real}$ is a value counting spikes with a spike interval (ISI) between two electrodes of 100 ms or less (synchronized spikes). Specifically, this is the total number of those with a difference between the time at which a spike is detected with the first electrode and time at which the spike is detected with the second electrode of 100 ms or less. $Ave_{surrogate}$ is the mean value of the number of synchronized spikes obtained by randomly rearranging detected spikes and counting those with ISI between two electrodes of 100 ms or less (synchronized spikes) in the rearranged data, and repeating this a plurality of times (e.g., 100 times). $SD_{surrogate}$ is the standard deviation of the number of synchronized spikes obtained by randomly rearranging detected spikes and counting those with ISI between two electrodes of 100 ms or less (synchronized spikes) in the rearranged data, and repeating this a plurality of times (e.g., 100 times). Since Z score is an indicator of synaptic connection strength, multivariate analysis including a Z score enables prediction of a property of a target compound which takes into consideration the effect on synaptic connection strength.

A more specific methodology for computing a Z score is described in, for example, Sasaki T, Suzuki I, Yokoi R, Sato K, Ikegaya Y. "Synchronous spike patterns in differently mixed cultures of human iPSC-derived glutamatergic and GABAergic neurons." Biochem Biophys Res Commun. 2019 May 28; 513(2): 300-305. doi: 10.1016/j.bbrc.2019.03.161. Epub 2019 Apr 4. This document is incorporated herein by reference.

A candidate parameter can comprise, for example, the following parameters.

*Normalized Duration IQR (interquartile range of burst duration normalized with a median value)
*Full Width at Half Height of Normalized Cross Correlation (half width of Cross-Correlogram)

A candidate parameter set can comprise all of the parameters described above, or some of the parameters described above. It can be preferable for a candidate parameter set to comprise at least one of the basic activity parameters, at least one of the burst structure parameters, at least one of the parameters related to periodicity, at least one of the parameters for analyzing a frequency, and at least one of the nonlinear time series analysis parameters. This is because this enables analysis comprising spike information, information on a synaptic current component from frequency analysis, and time series information on spike occurrence from nonlinear time series analysis. It can be still more preferable for a candidate parameter set to comprise all of the candidate parameter sets described above because this enables prediction of a property of a target compound with high accuracy.

Referring back again to Figure **2,** the prediction parameter identification means **122** is configured to identify a prediction parameter set based on a result of multivariate analysis by the multivariate analysis means **121.** For example, the prediction parameter identification means **122** identifies a combination of candidate parameters capable of isolating a first known compound and a second known compound as a prediction parameter set, based on a result of multivariate analysis. For example, the prediction parameter identification means **122** can also be configured to identify a combination of candidate parameters capable of isolating three or more compounds as a prediction parameter set based on a result of multivariate analysis.

If, for example, multivariate analysis is principle component analysis, a certain combination of candidate parameters can be identified as a prediction parameter set when a significant difference is found from performing a significance test on a first principle component score of activity data for a neuron in response to a first known compound and a first principle component score of activity data for a neuron in response to a second known compound from a result of principle component analysis on the combination of candidate parameters. For example, a certain combination of candidate parameters can be identified as a prediction parameter set when a significant difference is found from performing a significance test on the first principle component score of activity data for a neuron in response to the first known compound, the first principle component score of activity data for a neuron in response to the second known compound ... first principle component score of activity data for a neuron in response to the nth known compound from a result of principle component analysis on the combination of candidate parameters. Such a significance test is performed for every combinations of candidate parameters subjected to multivariate analysis, and one or more combinations of candidate parameters found to have a significant difference is identified as a prediction parameter set.

The prediction parameter identification means **122** can perform a significance test using any significance test methodology. A significance test can be performed using, for example, ANOVA (analysis of variance), or MANOVA (multivariate analysis of variance) for a multivariate test. For example, a significant difference can be found if ANOVA (analysis of variance) or MANOVA (multivariate analysis of variance) is performed and the resulting p value is 0.05 or less. The threshold value of p value at which a significant difference is found is not limited to 0.05. The threshold value can be any value.

If, for example, multivariate analysis is cluster analysis, a certain combination of candidate parameters can be identified as a prediction parameter set when activity data for a neuron in response to a first known compound and activity data for a neuron in response to a second known compound are each classified into separate clusters as a result of cluster analysis for the combination of candidate parameters. For example, a certain combination of candidate parameters can be identified as a prediction parameter set when activity data for a neuron in response to the first known compound, activity data for a neuron in response to the second known compound ... activity data for a neuron in response to the nth known compound are all classified into separate clusters as a result of cluster analysis for the combination of candidate parameters. In this manner, cluster analysis is performed for every combination of candidate parameters, and one or more combinations of candidate parameters whose entire data is classified into separate clusters is identified as a prediction parameter set.

It can be preferable for the prediction parameter identification means **122** to identify a plurality of prediction parameter sets capable of isolating each of a plurality of sets of compound. This is because this enables prediction of a plurality of properties. It can be still more preferable for the prediction parameter identification means **122** to identify a plurality of prediction parameter sets capable of isolating each of a plurality of associated sets of compound. This is because isolation of a plurality of associated sets of compound in stages enable prediction of a property in stages and prediction of a property with high accuracy.

The prediction means **123** is configured to predict a property from inputted data based on a prediction parameter set identified by the prediction parameter identification means **122**. The prediction means **123** receives a prediction parameter set from the prediction parameter identification means **122** and receives data for a target compound received by the receiving means **110**. Data for a target compound can be, for example, activity data for a neuron in response to a target compound or image data converted therefrom (e.g., raster plot image, histogram, waveform image, or the like).

For example, the prediction means **123** determines which of data for a first known compound and data for a second known compound the data for a target compound is similar to, and predicts the determined property of the first known compound or second known compound as the property of the target compound.

For example, the prediction means **123** can perform multivariate analysis on data for a target compound for a prediction parameter set and determine which data the data for the target compound is similar to based on the result thereof. If multivariate analysis is, for example, principle component analysis, the prediction means **123** computes a principle component score of data for a target compound for a prediction parameter set and determines whether it is similar based on a principle component plot that plots principle component scores. For example, similarity is determined by the Euclidean distance, cosine similarity, or a combination thereof between principle component plots. If multivariate analysis is, for example, cluster analysis, the prediction means **123** performs cluster analysis on data for a first known compound, data for a second known compound, and data for a target compound for a prediction parameter set and classifies each of the data for the first known compound, data for the second known compound, and data for the target compound into clusters. Similarity is determined based on whether the data for the target compound is classified into the same cluster as the data for the first known compound or the data for the second known compound. In this regard, if the data for the target compound is not classified to neither the cluster to which the data for the first known compound is classified nor the cluster to which the data for the second known compound is classified, the data for the target compound would be similar to neither the data for the first known compound nor the data for the second known compound.

For example, the prediction means **123** can determine which of the data for the first known compound and the data for the second known compound the data for the target compound is similar to by utilizing support vector machine (SVM). For example, the prediction means **123** can utilize SVM, for a prediction parameter set, to deduce a hyperplane dividing the data for the first known compound and the data for the second known compound and decide whether the data for the target compound belongs to the side of data for the first known compound or the side of data for the second known compound to determine which of data for the first known compound and data for the second known compound the data for the target compound is similar to.

The aforementioned example described that a processor comprises the multivariate analysis means **121** and the prediction parameter identification means **122,** but a processor does not need to comprise the multivariate analysis means **121** and the prediction parameter identification means **122** if a prediction parameter set is predetermined. In such a case, a processor receives a predetermined prediction parameter set to predict a property based on the received prediction parameter set.

For example, a processor performs multivariate analysis on data for a target compound, data for a first known compound, and data for a second known compound, for a predetermined prediction parameter set, and determines which of the data for the first known compound and the data for the second known compound the data for the target compound is similar to based on the result thereof to predict a determined property of the first known compound or second known compound as the property of the target compound. For example, a processor performs principle component analysis on data for a target compound, data for a first known compound, and data for a second known compound, for a predetermined prediction parameter set, and determines which of the data for the first known compound and the data for the second known compound the data is similar to based on a principle component plot (e.g., based on similarity S) to predict a determined property of the first known compound or second known compound as a property of the target compound. For example, a processor performs cluster analysis on data for a target compound, data for a first known compound, and data for a second known compound, for a predetermined prediction parameter set, and determines which of the data for the first known compound and the data for the second known compound the data is similar to, based on whether the data for the target compound is classified into the same cluster as the data for the first known compound or the data for the second known compound, to predict a determined property of the first known compound or second known compound as a property of the target compound. For example, SVM can be utilized to deduce a hyperplane dividing the data for the first known compound and the data for the second known compound, for a predetermined prediction parameter set, and decide whether the data for the target compound belongs to the side of data for the first known compound or the side of data for the second known compound to determine which of the data for the first known compound and the data for the second known compound the data for the target compound is similar to.

In the example shown in Figure **1,** each constituent element of the computer system **100** is provided within the computer system **100,** but the present invention is not limited thereto. Any of the constituent elements of the computer system **100** can also be provided external to the computer system **100.** If, for example, each of the processor **120** and memory **130** is comprised of separate hardware parts, each hardware part can be connected via any network. The type of such a network is not limited. Each hardware part can be connected, for example, via LAN, wirelessly, or with a wire. The computer system **100** is not limited to a specific hardware configuration. For example, the processor **120** comprised of an analog circuit instead of a digital circuit is also within the scope of the invention. The configuration of the computer system **100** is not limited to those described above, as long as the function thereof can be materialized.

In the example shown in Figure **2,** each constituent element of the processor **120** is provided within the same processor **120,** but the present invention is not limited thereto. A configuration with each constituent element of the processor **120** dispersed in a plurality of processor units is also within the scope of the invention.

3. Processing in a computer system for predicting a property of a target compound

Figure **7** shows an example of processing in the computer system **100** for predicting a property of a target compound. The example shown in Figure **7** describes processing **700** for predicting a property of a target compound.

At step **S701,** the computer system **100** receives first activity data for a neuron in response to a first known compound via the receiving means **110.** The first activity data can comprise, for example, a plurality of pieces of data from administering the first known compound to different cell species, a plurality of pieces of data from administering the first known compound to the same cell species sample supplied by different vendors, a plurality of pieces of data from administering the first known compound to samples of different lots supplied by the same vendor, a plurality of pieces of data from administering the first known compound to samples supplied on different dates by the same vendor, or a plurality of pieces of data from testing the first known compound at different facilities. The received activity data is passed along to the processor **120.**

At step **S702,** the computer system **100** receives second activity data for a neuron in response to a second known compound via the receiving means **110.** The second activity data can comprise, for example, a plurality of pieces of data from administering the second known compound to different cell species, a plurality of pieces of data from administering the second known compound to the same cell species sample supplied by different vendors, a plurality of pieces of data from administering the second known compound to samples of different lots supplied by the same vendor, a plurality of pieces of data from administering the second known compound to samples supplied on different dates by the same vendor, or a plurality of pieces of data from testing the second known compound at different facilities. The received activity data is passed along to the processor **120.**

At step **S703,** the processor **120** identifies a prediction parameter set by performing multivariate analysis on the first activity data and the second activity data for every combination of each of the candidate parameters in a candidate parameter set. For example, the multivariate analysis means **121** of the processor **120** performs multivariate analysis on the first activity data and the second activity data for every combination of each of the candidate parameters in a candidate parameter set, and the prediction parameter identification means **122** of the processor **120** identifies a prediction parameter set based on a result of the multivariate analysis.

When a prediction parameter set is identified and the computer system **100** receives target activity data for a neuron in response to a target compound via the receiving means **110,** at step **S704,** the processor **120** predicts a property based on the prediction parameter set identified at step **S703** from the target activity data for a neuron in response to the target compound. For example, the prediction means **123** of the processor **120** predicts a property. The property of the target compound can be predicted in this manner.

For example, when a plurality of pieces of first activity data are received at step **S701** described above, processing can be configured to perform multivariate analysis using a parameter capable of isolating a known property of a first known compound on each of the plurality of pieces of first activity data and pass long only the first activity data with the same property as the known property of the first known compound to the processor **120** for subsequent processing. This is performed, for example, by determining whether the first activity data has the same property as the known property of the first known compound and excluding the data, if the data does not have the same property, as an outlier value from data passed along to the processor **120.** The accuracy of prediction by the processor **120** is further improved by detecting an outlier value and excluding data comprising the outlier value from data passed along to the processor **120.** The aforementioned step **S702** can similarly detect an outlier value of second activity data and exclude data comprising the outlier value from data passed along to the processor **120.** The aforementioned step **S704** can similarly detect an outlier value of target activity data for a neuron in response to a target compound and exclude data comprising the outlier value from data passed along to the processor **120.**

The step of detecting an outlier value by multivariate analysis described above can also be utilized in other applications. The step is effective in, for example, lot difference check, quality check, or selection of a cell from different cell species in the manufacture of cells or the like. For example, an outlier value can be detected by performing multivariate analysis using a parameter capable of isolating a given property on cells of each of a plurality of lots and determining whether the cells have the same property as the given property in the manufacture of cells. This enables exclusion of a lot with a significant inter-lot difference. For example, an outlier value can be detected by performing multivariate analysis using a parameter capable of isolating a desired quality on each of a plurality of cells and determining whether the cells have the same quality as the desired quality in the manufacture of cells. This enables exclusion of cells without the desired quality. When selecting, for example, a cell from different cell species, an outlier value can be detected by performing multivariate analysis using a parameter capable of isolating a desired property on cells of each of a plurality of cell species and determining whether the cells have the same property as the desired property. This enables exclusion of cell species without the desired property.

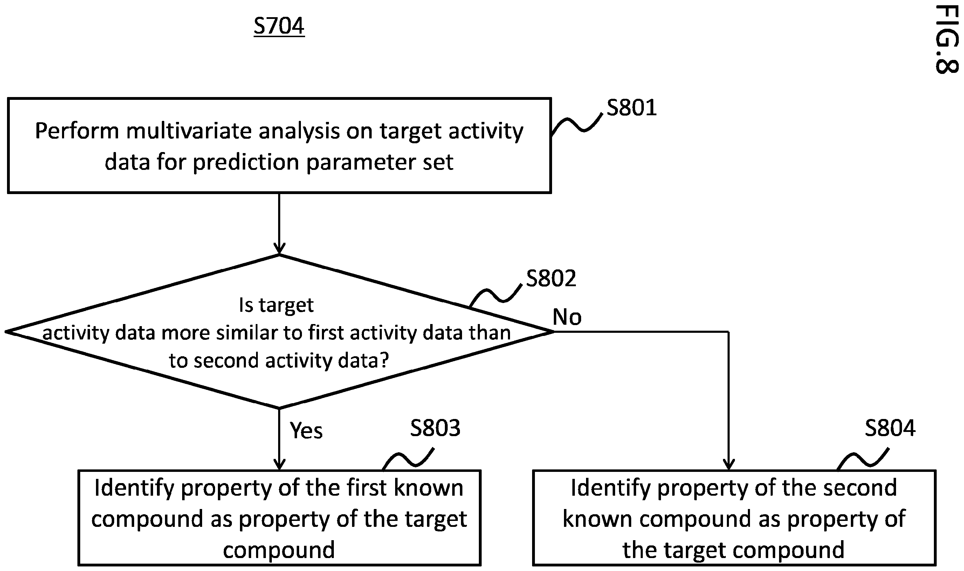

Figure **8** shows an example of processing to predict a property of a target compound by the processor **120** at step **S704.**

At step **S801,** the prediction means **123** of the processor **120** performs multivariate analysis on target activity data with respect to a target compound for a prediction parameter set identified at step **S703.** This enables comparison of the target activity data with a result of multivariate analysis for a prediction parameter set already performed on first activity data and second activity data. Multivariate analysis is, for example, principle component analysis. For example, the prediction means **123** computes a principle component score of target activity data. Multivariate analysis is, for example, cluster analysis. For example, the prediction means **123** classifies target activity data into a cluster by cluster analysis.

The multivariate analysis performed at step **S801** can use the same methodology or different methodology as the multivariate analysis performed at step **S703.** If, for example, a prediction parameter set is identified by principle component analysis at step **S703,** principle component analysis can be performed in the same manner or different multivariate analysis (e.g., cluster analysis) can be performed at step **S801.** If, for example, a prediction parameter set is identified by cluster analysis at step **S703,** cluster analysis can be performed in the same manner or different multivariate analysis (e.g., principle component analysis) can be performed at step **S801.**

For example, the prediction means **123** of the processor **120** can, at step **S801,** deduce a hyperplane dividing first activity data and second activity data by utilizing SVM, for a prediction parameter set, and determine whether target activity data for a target compound belongs to the side of the first activity data of the hyperplane or the side of the second activity data of the hyperplane.

At step **S802,** the prediction means **123** of the processor **120** determines whether the target activity data is more similar to the first activity data than to the second activity data.

For example, the prediction means **123** can determine whether data is similar based on a principle component score computed at step **S801.** For example, similarity of the target activity data to the first activity data and similarity of the target activity data to the second activity data can be computed based on a principle component plot created based on a principle component score.

For example, it can be determined whether target activity data is more similar to first activity data than to second activity data by plotting a first principle component score and second principle component score of the first activity data, plotting a first principle component score and second principle component score of the second activity data, and plotting a first principle component score and second principle component score of the target activity data for a prediction parameter set, and then comparing the Euclidean distance between the plot of the target activity data and the plot of the first activity data with the Euclidean distance between the plot of the target activity data and the plot of the second activity data. For example, it can be determined whether target activity data is more similar to first activity data than to second activity data by comparing cosine similarity between the plot of the target activity data and the plot of the first activity data with cosine similarity between the plot of the target activity data and the plot of the second activity data. For example, it can be determined whether target activity data is more similar to first activity data than to second activity data by comparing the Euclidean distance and cosine similarity between the plot of the target activity data and the plot of the first activity data with the Euclidean distance and cosine similarity between the plot of the target activity data and the plot of the second activity data. When a plurality of points are plotted with a plurality of pieces of first activity data or a plurality of pieces of second activity data, the mean value of all plots can be computed and used as a representative point to find the Euclidean distance or cosine similarity with respect to target activity data.

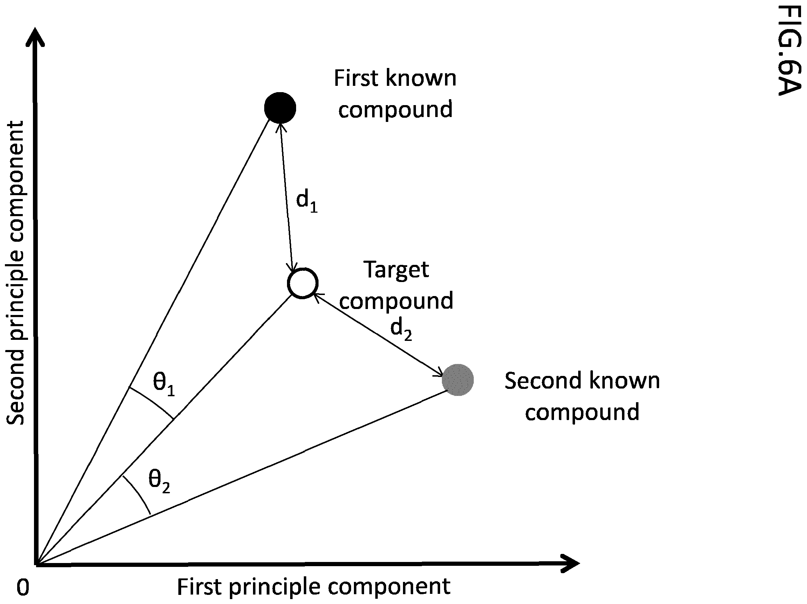

Preferably, similarity is determined by a combination of the Euclidean distance and cosine similarity of principle component plots. This is because, for example, even if the Euclidean distance $d_1$ between a plot of a target compound (target activity data) and a plot of a first known compound (first activity data) is equal to Euclidean distance $d_2$ between the plot of the target compound (target activity data) and a plot of a second known compound (second activity data) as shown in Figure **6A,** similarly can be distinguished by cosine similarity. When a combination of Euclidean distance and cosine similarity between principle component plots is used, similarity S is represented by

[Numeral 5]

$$S = \frac{cos\,\vartheta}{d}$$

For example, the prediction means **123** can determine whether target activity data is more similar to first activity data than to second activity data based on which cluster the target activity data is classified into at step **S801**.

The prediction means **123** can determine whether target activity data is more similar to first activity data than to second activity data based on, for example, which of the side of the first activity data and the side of the second activity data of a hyperplane deduced by SVM the target activity data of a target compound is determined to belong to at step **S801**. If the target activity data belongs to the side of the first activity data of the hyperplane, the target activity data is determined to be more similar to the first activity data than to the second activity data. If the target activity data belongs to the side of the second activity data of the hyperplane, the target activity data is determined to be more similar to the second activity data than to the first activity data.

If the target activity data is determined to be more similar to the first activity data than to the second activity data at step **S802,** the process proceeds to step **S803** and a property of a first known compound is identified as a property of a target compound. If, for example, the first known compound has a property of "with toxicity", the property of the target compound is identified as "with toxicity". If, for example, the first known compound has a property (mechanism of action) of "effective on GABA", the mechanism of action of the target compound is identified as "effective on GABA".

If the target activity data is determined to be not more similar to the first activity data than to the second activity data, i.e., target activity data is determined to be more similar to the second activity data than to the first activity data at step **S802,** the process proceeds to step **S804** and identifies the property of the second known compound as a property of the target compound. If, for example, the second known compound has a property of "without toxicity", the property of the target compound is identified as "without toxicity". If, for example, the second known compound has a property (mechanism of action) of "effective on K channel", the mechanism of action of the target compound is identified as "effective on K channel".

The aforementioned example performed processing **700** using the first activity data for the first known compound and the second activity data for the second known compound, but the processing **700** can also be configured to be performed by additionally using third activity data for a third known compound, fourth activity data for a fourth known compound ... nth activity data for an nth compound (wherein n is an integer that is 3 or greater) . In this regard, similarity of target activity data with each of the first activity data, second activity data ... nth activity data is compared at step **S802.**

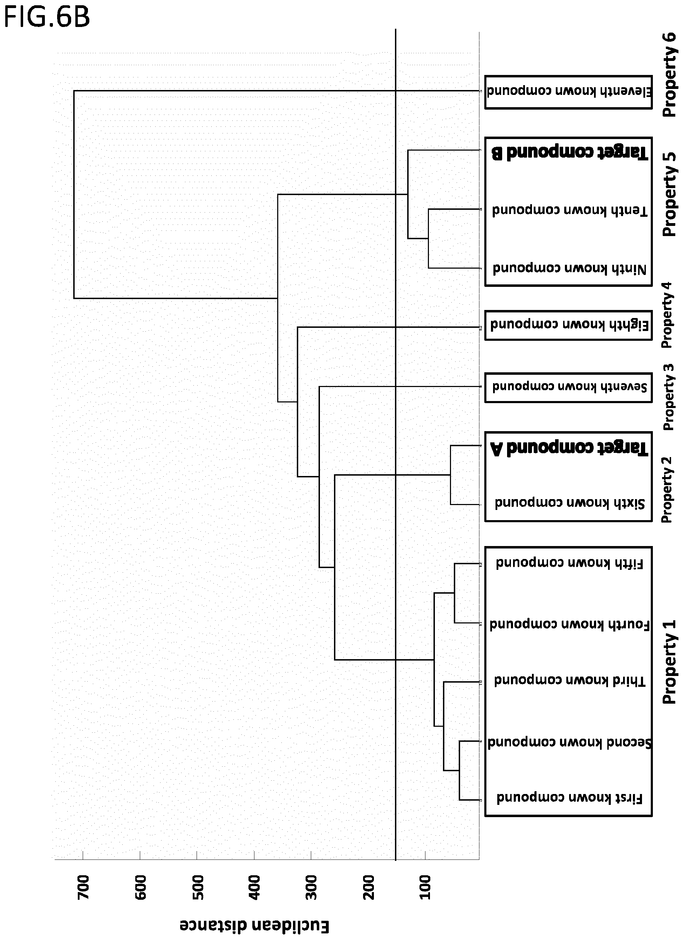

The processing **700** can be performed by using, for example, first activity data for a first known compound, second activity data for a second known compound ... eleventh activity data for an eleventh known compound. Figure **6B** shows an example of a result when cluster analysis was performed as multivariate analysis at step **S801** in such a case.

Figure **6B** shows an example of a result of performing cluster analysis on first to eleventh known compounds and target compound A and target compound B. The vertical axis indicates the Euclidean distance. In Figure **6B,** compounds with a close Euclidean distance (e.g., compounds with a Euclidean distance of about 150 (shown as a two dot chain line in Figure **6B)** or less) are classified into the same cluster, and compounds within the same cluster have the same property. For example, first to fifth known compounds each have the same property 1, sixth known compound has property 2, seventh known compound has property 3, eighth known compound has property 4, ninth and tenth known compounds each have the same property 5, and eleventh known compound has property 6. In this regard, target compound A is classified into the same cluster as the sixth known compound, so that the compound is predicted to have property 2. Since target compound B is classified into the same cluster as the ninth and tenth known compounds, the compound is predicted to have property 5.

After identifying the property of the target compound at step **S803** or step **S804,** step **S704** can be repeated using a second prediction parameter set. In this regard, the second prediction parameter set can be, for example, a prediction parameter set identified by performing processing of steps **S701** to **S703** using a third known compound and fourth known compound. Alternatively, the second prediction parameter set can be a prediction parameter set identified by performing the processing of steps **S701** to **S703** using a first known compound and third known compound if the property of the first known compound is identified as the property of the target compound at step **S803,** or a prediction parameter set identified by performing the processing of steps **S701** to step **S703** using the second known compound and third known compound if the property of the second known compound is identified as the property of the target compound at step **S804.** The second prediction parameter would correspond to the property of the target compound identified at step **S803** or **S804** thereby. This can be preferable in terms of being able to identify a property in stages.

If, for example, the property of the target compound is identified as "effective on GABA" at step **S803** or **S804,** step **S704** can be repeated using the second prediction parameter identified by performing the processing of steps **S701** to **S703** using the third known compound having the property (mechanism of action) of "effective on GABA-A" and the first known compound or fourth known compound having the property (mechanism of action) of "effective on GABA-B". This enables identification of a specific property, as to whether the property of the target compound of "effective on GABA" is "effective on GABA-A" or "effective on GABA-B".

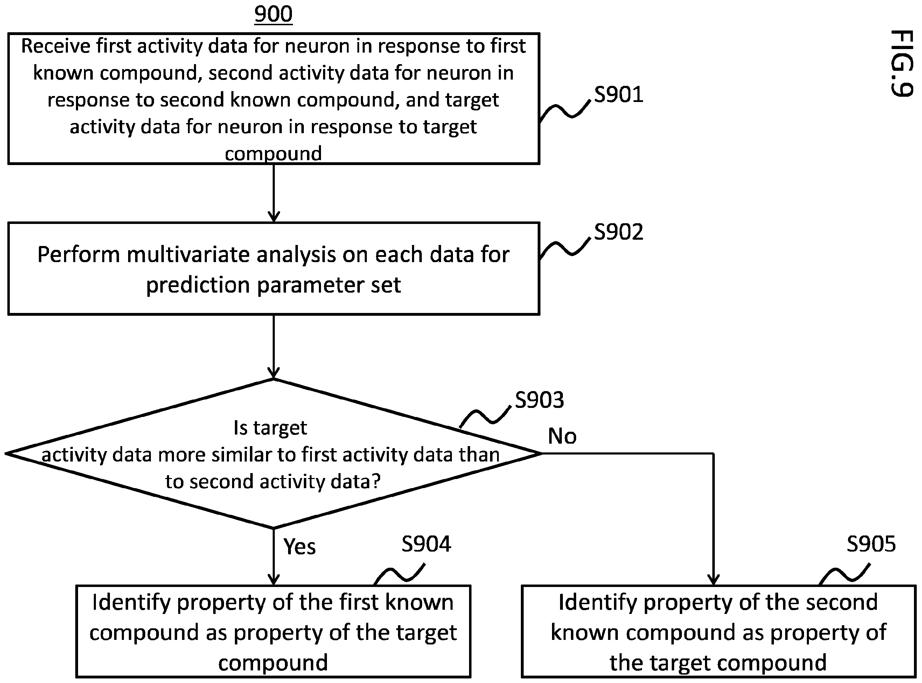

Figure **9** shows an example of processing in the computer system **100** for predicting a property of a target compound. The example shown in Figure **7** describes processing **900** for predicting a property of a target compound when a prediction parameter set is predetermined. A predetermined prediction parameter set comprises at least one of the candidate parameters described above.

At step **S901,** the computer system **100** receives first activity data for a neuron in response to a first known compound, second activity data for a neuron in response to a second known compound, and target activity data for a neuron in response to a target compound via the receiving means **110.** The first activity data can comprise, for example, a plurality of pieces of data from administering the first known compound to different cell species, a plurality of pieces of data from administering the first known compound to the same cell species sample supplied by different vendors, a plurality of pieces of data from administering the first known compound to samples of different lots supplied by the same vendor, a plurality of pieces of data from administering the first known compound to samples supplied on different dates by the same vendor, or a plurality of pieces of data from testing the first known compound at different facilities. The second activity data can comprise, for example, a plurality of pieces of data from administering the second known compound to different cell species, a plurality of pieces of data from administering the second known compound to the same cell species sample supplied by different vendors, a plurality of pieces of data from administering the second known compound to samples of different lots supplied by the same vendor, a plurality of pieces of data from administering the second known compound to samples supplied on different dates by the same vendor, or a plurality of pieces of data from testing the second known compound at different facilities. Received activity data is passed along to the processor **120.**

For example, when a plurality of pieces of first activity data are received at step **S901** described above, processing can be configured to perform multivariate analysis using a parameter capable of isolating a known property of a first known compound on each of the plurality of pieces of first activity data and pass long only the first activity data with the same property as the known property of the first known compound to the processor **120** for subsequent processing. This is performed, for example, by determining whether the first activity data has the same property as the known property of the first known compound and excluding the data, if the data does not have the same property, as an outlier value from data passed along to the processor **120.** The accuracy of prediction by the processor **120** is further improved by detecting an outlier value and excluding data comprising the outlier value from data passed along to the processor **120.** An outlier value can be similarly detected for the second activity data and target activity data for a neuron in response to a target compound to exclude data comprising the outlier value from data passed along to the processor **120.**

At step **S902,** the prediction means **123** of the processor **120** performs multivariate analysis for a prediction parameter set on each data. Multivariate analysis is, for example, principle component analysis. For example, the prediction means **123** computes a principle component score of first activity data, principle component score of second activity data, and principle component score of target activity data by principle component analysis. Multivariate analysis is, for example, cluster analysis. For example, the prediction means **123** classifies target activity data into a cluster by cluster analysis.

For example, the prediction means **123** of the processor **120** can, at step **S902,** deduce a hyperplane dividing first activity data and second activity data by utilizing SVM, for a prediction parameter set, to determine whether target activity data for a target compound belongs to the side of the first activity data of the hyperplane or the side of the second activity data of the hyperplane.

At step **S903,** the prediction means **123** of the processor **120** determines whether the target activity data is more similar to the first activity data than to the second activity data. For example, the prediction means **123** can determine whether the data is similar based on a principle component score computed at step **S902.** For example, similarity of the target activity data to the first activity data and similarity of the target activity data to the second activity data can be computed based on a principle component plot created based on a principle component score.

For example, it can be determined whether target activity data is more similar to first activity data than to second activity data by plotting a first principle component score and second principle component score of the first activity data, plotting a first principle component score and second principle component score of the second activity data, and plotting a first principle component score and second principle component score of the target activity data, for a prediction parameter set, and then comparing the Euclidean distance between the plot of the target activity data and the plot of the first activity data with the Euclidean distance between the plot of the target activity data and the plot of the second activity data. For example, it can be determined whether target activity data is more similar to first activity data than to second activity data by comparing cosine similarity between the plot of the target activity data and the plot of the first activity data with cosine similarity between the plot of the target activity data with the plot of the second activity data. For example, it can be determined whether target activity data is more similar to first activity data than to second activity data by comparing the Euclidean distance and cosine similarity between the plot of the target activity data and the plot of the first activity data with the Euclidean distance and cosine similarity between the plot of the target activity data and the plot of the second activity data. When a plurality of points are plotted with a plurality of pieces of first activity data or a plurality of pieces of second activity data, the mean value of all plots can be computed and used as a representative point to find the Euclidean distance or cosine similarity with respect to target activity data.

For example, the prediction means **123** can determine whether target activity data is more similar to first activity data than to second activity data based on which cluster the target activity data is classified into at step **S902.**

The prediction means **123** can determined whether target activity data is more similar to first activity data than to second activity data based on, for example, which of the side of the first activity data or the side of the second activity data of a hyperplane deduced by SVM the target activity data of a target compound is determined to belong to at step **S902.** If the target activity data belongs to the side of the first activity data of the hyperplane, the target activity data is determined to be more similar to the first activity data than to the second activity data. If the target activity data belongs to the side of the second activity data of the hyperplane, the target activity data is determined to be more similar to the second activity data than to the first activity data.

If the target activity data is determined to be more similar to the first activity data than to the second activity data at step **S903,** the process proceeds to step **S904,** and a property of a first known compound is identified as a property of a target compound. If, for example, the first known compound has a property of "with toxicity", the property of the target compound is identified as "with toxicity". If, for example, the first known compound has a property (mechanism of action) of "effective on GABA", the mechanism of action of the target compound is identified as "effective on GABA".

If the target activity data is determined to be not more similar to the first activity data than to the second activity data, i.e., target activity data is determined to be more similar to the second activity data than to the first activity data at step **S903,** the process proceeds to step **S905** and identifies a property of a second known compound as a property of a target compound. If, for example, the first known compound has a property of "without toxicity", the property of the target compound is identified as "without toxicity". If, for example, the second known compound has a property (mechanism of action) of "effective on K channel", the mechanism of action of the target compound is identified as "effective on K channel".

The aforementioned example performed processing **900** using the first activity data for the first known compound and the second activity data for the second known compound, but the processing **900** can also be performed by additionally using third activity data for a third known compound, fourth activity data for a fourth known compound ... nth activity data for an nth compound (wherein n is an integer that is 3 or greater). In this regard, similarity of target activity data to the first activity data, second activity data ... nth activity data is compared at step **S903.**

In the example described above, processing was described as being performed in a specific order, but it should be noted that the order of each processing is not limited to the described order, and is performed in any theoretically feasible order.

The aforementioned example described prediction of a property of a target compound using the computer system **100,** but the subject that can be predicted is not limited to a property of a compound. A computer system capable of predicting a property of any target is also within scope of the invention, as long as there is a prediction parameter set capable of isolating the property.

For example, the present invention can target a neural network as a subject, and a property of an unknown neural network can be predicted with the computer system of the invention. A neural network can be, for example, a neural network prepared from an iPS cell.

For example, multivariate analysis on electrical activity data for a known disease neuron is performed for every combination of each of the candidate parameters in a candidate parameter set (can be the same candidate parameter set as the candidate parameter set used in predicting a property of a compound) to identify a prediction parameter set capable of isolating electrical activity data for different disease neurons. This can predict the disease neuron to which the function of an unknown neural network is close, based on the identified prediction parameter set. This can identify, for example, the disease neuron whose electrical activity is similar to the electrical activity exhibited by an unknown neural network prepared from a healthy individual, thus can enable prediction of a future disease of the healthy individual.

For example, multivariate analysis on electric activity data for a known neural network at each part of the brain is performed for every combination of each of the candidate parameters in a candidate parameter set (can be the same candidate parameter set as the candidate parameter set used in predicting a property of a compound) to identify a prediction parameter set capable of isolating electrical activity data for a neural network at each part of the brain. This can predict the part of the brain which has a behavior close to the unknown neural network, based on the identified prediction parameter set.

In the aforementioned example, the accuracy of prediction can be improved by using data such as gene expression data, synaptic function data, or metabolic function data in addition to electrical activity data. A property that can be isolated can also be increased.

For example, the present invention can target a cell as a subject and predict a property of an unknown cell with the computer system of the invention.

For example, multivariate analysis on various data (e.g., gene expression data, autophagy data, mitochondria analysis data, senescent cell analysis data, or the like) obtained from cells of patients with a known disease is performed for every combination of each of the candidate parameters in a candidate parameter set (can be a candidate parameter set comprising a parameter for quantitative data (e.g., quantitative data for gene expression, autophagy, mitochondria, or senescent cells) from various cell attribute analysis) to identify a prediction parameter set capable of isolating cell data for patients with different known diseases. This can predict the cell of a patient with a known disease which has a property close to the cell of a patient with an unknown disease, based on the identified prediction parameter set, thus enabling, for example, diagnosis of a disease or prediction of illness of a patient with an unknown disease.

Such a computer system that can predict a property of any target can have the same configuration as the computer system **100**. A property of any target can be predicted by the same processing as that of the computer system **100**. For example, a property of any target can be predicted by the same processing as the processing **700** described above.

At step **S701,** the computer system **100** receives first data for a first subject via the receiving means **110.** The first subject can be, for example, a first known disease neuron, a neural network of a first part of the brain, cell of a first patient with a known disease, or the like. The first data can be electrical activity data for the first known disease neuron, electrical activity data for the neural network of the first part of the brain, and various data (e.g., gene expression data, autophagy data, mitochondria analysis data, senescent cell analysis data, or the like) for the cell of the first patient with a known disease, respectively. The received data is passed along to the processor **120.**

At step **S702,** the computer system **100** receives second data for a second subject via the receiving means **110.** The second subject can be, for example, a second known disease neuron, a neural network of a second part of the brain, cell of a second patient with a known disease, or the like. The second data can be electrical activity data for the second known disease neuron, electrical activity data for the neural network of the second part of the brain, and various data (e.g., gene expression data, autophagy data, mitochondria analysis data, senescent cell analysis data, or the like) for the cell of the second patient with a known disease, respectively. The received activity data is passed along to the processor **120.**

A step **S703,** the processor **120** identifies a prediction parameter set by performing multivariate analysis on first data and second data for every combination of each of the candidate parameters in a candidate parameter set. For example, the multivariate analysis means **121** of the processor **120** performs multivariate analysis on first data and second data for every combination of each of the candidate parameters in a candidate parameter set, and the prediction parameter identification means **122** of the processor **120** identifies a prediction parameter set based on the result of the multivariate analysis.

Once a prediction parameter set is identified and the computer system **100** receives target data for a target via the receiving means **110,** at step **S704,** the processor **120** predicts a property based on the prediction parameter set identified at step **S703** from the target data for a target. For example, the prediction means **123** of the processor **120** predicts a property. The target can be, for example, an unknown neural network, a cell of a patient with an unknown disease, or the like, and the target disease can be electrical activity data for the unknown neural network and various data (e.g., gene expression data, autophagy data, mitochondria analysis data, senescent cell analysis data, or the like) for the cell of a patient with an unknown disease, respectively. A property of any target can be predicted in this manner.

In the examples described above in reference to Figures **7, 8,** and **9,** processing in each step shown in Figures **7, 8,** and **9** is described to be materialized with the processor **120** and a program stored in the memory **130,** but the present invention is not limited thereto. At least one of the processing of each step shown in Figures **7, 8,** and **9** can be materialized with a hardware configuration such as a control circuit.

The aforementioned example described prediction of a property of a subject using the computer system **100,** but the present invention is not limited thereto. Prediction of a property of a subject through manual calculation by a person without using the computer system **100** is also within the scope of the invention. In such a case, the processing **700** and **900** can comprise, for example, a step of obtaining data from a subject instead of receiving data (step **S701, S702,** or step **S901).** Subsequent steps can be performed by manual calculation based on the obtained data.

[Examples]

In one Example, activity data from administrating each of five known compounds (4AP, Phenytoin, PTX (Picrotoxin), Strychnine, and Amoxiciline) to a neuron was obtained using MEA. In this regard, Axol Bioscience's Human iPSC-derived neural stem cells (ax0019) were used as the neurons, Alpha MED Scientific's MEA 24-well Plate-comfort was used as MEA, and Alpha MED Scientific's MED64-Presto (MED-A384iN) was used as the measurement device thereof. 4AP was administered at a concentration of about 30 $\mu$M. Phenytoin was administered at a concentration of about 100 $\mu$M. PTX (Picrotoxin) was administered at a concentration of about 3 $\mu$M. Strychnine was administered at a concentration of about 30 $\mu$M. Amoxiciline was administered at a concentration of about 100 $\mu$M. The obtained data was inputted into the computer system of the invention to perform principle component analysis on the data for every combination of each of the candidate parameters in a candidate parameter set. The candidate parameter set consisted of 10 parameters ((1) TS, (2) NoB, (3) IBI, (4) burst duration, (5) spike count in a burst, (6) PS, (7) coefficient of variation of PS, (8) IPI, (9) coefficient of variation of IPI, and (10) periodicity). Principle component analysis was performed for each of 968 parameter sets comprised of a combination of 3 or more of 10 parameters. As a result of principle component analysis and significance test, a parameter set consisting of IBI, spike count in a burst, PS, and periodicity was identified as a prediction parameter set capable of isolating the mechanism of action of 5 known compounds.

Principle component analysis was performed using a parameter set identified for activity data obtained from target compound A, with a compound having the same mechanism of action as PTX (Picrotoxin) as target compound A. Ten runs were performed for target compound A and each of the known compounds.

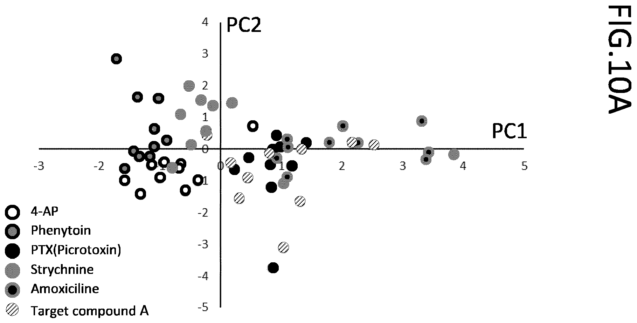

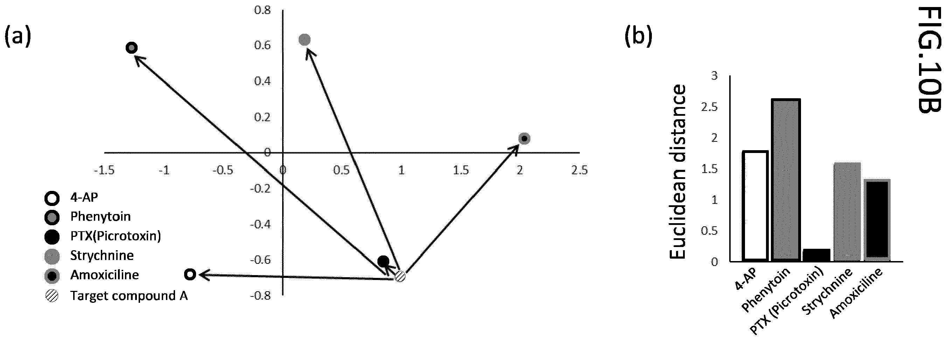

Figures **10A** and **10B** are diagrams showing a result of predicting a mechanism of action of target compound A in this Example.

Figure **10A** is a graph showing principle component plots of target compound A and five known compounds. In Figure **10A,** the horizontal axis indicates the first principle component, and the vertical axis indicates the second principle component.

Figure **10B(a)** is a graph showing the mean value of principle component plots of target compound A, the mean values of principle components plots of each of the five known compounds, and the Euclidean distance. Figure **10B(b)** shows each Euclidean distance with a bar graph. The leftmost bar graph shows the Euclidean distance between the mean value of principle component plots of target compound A and the mean value of principle component plots of 4AP. The second bar graph from the left shows the Euclidean distance between the mean value of principle component plots of target compound A and the mean value of principle component plots of Phenytoin. The third bar graph from the left shows the Euclidean distance between the mean value of principle component plots of target compound A and the mean value of principle component plots of PTX (Picrotoxin). The fourth bar graph from the left shows the Euclidean distance between the mean value of principle component plots of target compound A and the mean value of principle component plots of Strychnine. The fifth bar graph from the left shows the Euclidean distance between the mean value of principle component plots of target compound A and the mean value of principle component plots of Amoxiciline.

As can be seen from Figure **10B,** a known compound with the closest Euclidean distance from the mean value of the principle component plots of target compound A is PTX (Picrotoxin), so that the mechanism of action of target compound A is predicted to be the same as PTX (Picrotoxin). The mechanism of action of target compound A was able to be correctly predicted in this manner.

The present invention is not limited to the aforementioned embodiments. It is understood that the scope of the present invention should be interpreted solely from the scope of the claims. It is understood that those skilled in the art can implement an equivalent scope, based on the descriptions of the invention and common general knowledge, from the descriptions of the specific preferred embodiments of the invention.

[Industrial Applicability]

The present invention is useful as an invention providing a method of predicting an unknown property (e.g., toxicity to or efficacy on the nervous system) of a target such as a target compound and the like.

[Reference Signs List]

| | |
|---|---|
| **100** | Computer system |
| **110** | Receiving means |
| **120** | Processor |
| **130** | Memory |
| **140** | Outputting means |
| **200** | Database unit |

Claims

1. A method of predicting a property of a target, comprising the steps of:

   (1) obtaining first data for a first subject;
   (2) obtaining second data for a second subject;
   (3) identifying a prediction parameter set by performing multivariate analysis on the first data and the second data for every combination of each of candidate parameters in a candidate parameter set; and

(4) predicting the property based on the prediction parameter set from target data for the target.

2. The method of claim 1, wherein the step (3) for identifying the prediction parameter set comprises identifying a combination of the candidate parameters that can isolate the first subject from the second subject by the multivariate analysis as the prediction parameter set.

3. The method of claim 1 or 2, wherein the step (4) for predicting comprises comparing the target data with the first data and the second data to identify a property of a subject that yields data that is similar to the target data as a property of the target.

4. The method of claim 3, comprising determining which of the first data and the second data the target data is similar to by multivariate analysis on the target data, the first data, and the second data for the prediction parameter set.

5. The method of claim 4, wherein the similarity is determined by a Euclidian distance, cosine similarity, or a combination thereof.

6. The method of any one of claims 1 to 5, further comprising a step of:
(5) predicting a second property from second target data for the target based on a second prediction parameter set corresponding to the property predicted in the step (4).

7. The method of any one of claims 1 to 6, wherein the multivariate analysis is principle component analysis or cluster analysis.

8. The method of any one of claims 1 to 7, wherein the target is a compound.

9. The method of claim 8, wherein the property comprises one or more of efficacy, toxicity, and mechanism of action of the compound.

10. The method of claim 9, wherein the data is activity data for a neuron.

11. The method of claim 10, wherein the activity data is obtained using one of a micro-electrode array, $Ca^{2+}$ imaging, and membrane potential imaging.

12. The method of claim 10 or 11, wherein the neuron is a neural stem cell.

13. The method of claim 12, wherein the neural stem cell is an iPS cell.

14. The method of any one of claims 10 to 13, wherein the candidate parameter set comprises a basic activity parameter.

15. The method of claim 14, wherein the basic activity parameter comprises TS, NoB, a burst frequency, a burst percentage, and an interquartile range of a burst duration.

16. The method of any one of claims 10 to 15, wherein the candidate parameter set comprises a mean value of burst structure parameters.

17. The method of any one of claims 10 to 15, wherein the candidate parameter set comprises a standard deviation or a median absolute deviation of burst structure parameters.

18. The method of claim 16 or 17, wherein the burst structure parameters comprise a burst duration, a spike count in a burst, IBI, IPI, PS, peak time percentage, mean ISI within a burst, median ISI within a burst, and median/mean ISI within a burst.

19. The method of any one of claims 10 to 18, wherein the candidate parameter set further comprises a parameter related to periodicity.

20. The method of claim 19, wherein the parameter related to periodicity comprises a periodic parameter.

21. The method of claim 19 or 20, wherein the parameter related to periodicity further comprises coefficients of variation of each of a burst duration, a spike count in a burst, IBI, IPI, PS, peak time percentage, mean ISI within a burst, median ISI within a burst, and median/mean ISI within a burst and a mean value of CV ISI within a burst, standard deviation of CV ISI within a burst, median absolute deviation of CV ISI within a burst, and coefficients of variation of CV ISI within a burst.

22. The method of any one of claims 10 to 21, wherein the candidate parameter set comprises a parameter for analyzing a frequency.

23. The method of claim 22, wherein the parameter for analyzing a frequency comprises a parameter for analyzing a frequency of about 250 Hz or less.

24. The method of any one of claims 10 to 23, wherein the candidate parameter set comprises a nonlinear time series analysis parameter.

25. A method of predicting a property of a target, comprising the steps of:

obtaining data for each of a first subject, a second subject, and the target;
performing multivariate analysis on each data for a prediction parameter set;
determining which of the data for the first subject and the data for the second subject the data for the target is similar to; and
identifying a property of the first subject as the property of the target if the data for the target is determined to be more similar to the data for the first subject than to the data for the second subject.

26. The method of claim 25, wherein the prediction parameter set comprises one or more of a periodic parameter, a parameter for analyzing a frequency of about 250 Hz or less, and a nonlinear time series analysis parameter.

27. A computer system for predicting a property of a target, comprising:

means for receiving first data for a first subject and second data for a second subject;
means for performing multivariate analysis on the first data and the second data for every combination of each of candidate parameters in a candidate parameter set;
means for identifying a prediction parameter set based on a result of the multivariate analysis; and
means for predicting the property based on the prediction parameter set from target data for the target.

28. A program for predicting a property of a target, the program being executed in a computer system comprising a processor, the program causing the processor to execute processing comprising the steps of:

(1) receiving first data for a first subject;
(2) receiving second data for a second subject;
(3) identifying a prediction parameter set by performing multivariate analysis on the first data and the second data for every combination of each of candidate parameters in a candidate parameter set; and
(4) predicting the property based on the prediction parameter set from target data for the target.

29. A computer system for predicting a property of a target, comprising:

means for receiving data for each of a first subject, a second subject, and the target;
means for performing multivariate analysis on each data for a prediction parameter set;
means for determining which of the data for the first subject and the data for the second subject the data for the target is similar to; and
means for identifying a property of the first subject as the property of the target if the data for the target is determined to be more similar to the data for the first subject than to the data for the second subject.

30. A program for predicting a property of a target, the program being executed in a computer system comprising a processor, the program causing the processor to execute processing comprising the steps of:

receiving data for each of a first subject, a second subject, and the target;
performing multivariate analysis on each data for a prediction parameter set;
determining which of the data for the first subject and the data for the second subject the data for the target is similar to; and

identifying a property of the first subject as the property of the target if the data for the target is determined to be more similar to the data for the first subject than to the data for the second subject.

FIG.1

FIG.2

FIG.3

# FIG.4A

FIG.4B

## FIG.4C

FIG.5A

FIG.5B

## FIG.6A

# FIG.6B

# FIG.7

700

S701

Receive first activity data for neuron in response to first known compound

S702

Receive second activity data for neuron in response to second known compound

S703

Identify prediction parameter set by performing multivariate analysis on the first activity data and second activity data for every combination of each of the candidate parameters in a candidate parameter set

S704

Predict a property based on the prediction parameter set from target activity data for neuron in response to target compound

FIG.8

EP 3 859 329 A1

S704

| Perform multivariate analysis on target activity data for prediction parameter set | S801 |

Is target activity data more similar to first activity data than to second activity data? — S802

No

Yes — S803

S804

| Identify property of the first known compound as property of the target compound |

| Identify property of the second known compound as property of the target compound |

FIG.9

900

Receive first activity data for neuron in response to first known compound, second activity data for neuron in response to second known compound, and target activity data for neuron in response to target compound

～S901

↓

Perform multivariate analysis on each data for prediction parameter set

～S902

↓

Is target activity data more similar to first activity data than to second activity data?

～S903

No →

Yes ↓ S904

| Identify property of the first known compound as property of the target compound |

S905

| Identify property of the second known compound as property of the target compound |

# FIG.10A

# FIG.10B

## INTERNATIONAL SEARCH REPORT

| International application No. |
| --- |
| PCT/JP2019/038242 |

**A. CLASSIFICATION OF SUBJECT MATTER**
Int.Cl. G01N33/50(2006.01)i, G01N27/02(2006.01)i, G01N33/48(2006.01)i, G16B40/00(2019.01)i

According to International Patent Classification (IPC) or to both national classification and IPC

**B. FIELDS SEARCHED**

Minimum documentation searched (classification system followed by classification symbols)
Int.Cl. G01N27/00-27/10, 27/14-27/24, 33/48-33/98, G16B40/00-40/30

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2015-507470 A (COLD SPRING HARBOR LABORATORY, AN EDUCATION CORPORATION OF THE STATE OF NEW YORK) 12 March 2015, paragraphs [0002], [0005], [0235]-[0251], fig. 49-52 & US 2014/0297199 A1, paragraphs [0002], [0005], [0307]-[0335], fig. 49-52 & WO 2013/071099 A1 & CA 2854469 A1 & CN 104040719 A | 1-11, 14-30<br>12-13 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 26 November 2019 (26.11.2019) | 10 December 2019 (10.12.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2019/038242 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2013-13384 A (MEDICHROME KK) 24 January 2013, paragraphs [0001], [0015], [0031] (Family: none) | 12-13 |
| A | JP 2008-518598 A (NOVARTIS AG.) 05 June 2008 & US 2008/0096770 A1 & WO 2006/050124 A2 | 1-30 |
| A | JP 2005-502937 A (PSYCHOGENICS INC.) 27 January 2005 & US 2003/0004652 A1 & WO 2002/092101 A1 & EP 2246799 A1 & CA 2446853 A1 | 1-30 |
| A | US 2008/0118576 A1 (THEODORESCU et al.) 22 May 2008 & WO 2008/027912 A2 | 1-30 |
| A | WO 2017/091147 A1 (AGENCY FOR SCIENCE, TECHNOLOGY AND RESEARCH) 01 June 2017 & KR 10-2018-0086438 A & CN 108885204 A | 1-30 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. ODAWARA ; H. KATOH ; N. MATSUDA ; I. SU-ZUKI.** Physiological maturation and drug responses of human induced pluripotent stem cell-derived cortical neuronal networks in long-term culture. *Scientific Reports,* 2016, vol. 6

- **SASAKI T ; SUZUKI I ; YOKOI R ; SATO K ; IKE-GAYA Y.** Synchronous spike patterns in differently mixed cultures of human iPSC-derived glutamatergic and GABAergic neurons. *Biochem Biophys Res Commun.,* 28 May 2019, vol. 513 (2), 300-305